# EUROPEAN PATENT SPECIFICATION

(11) **EP 2 173 428 B1**
(45) Date of publication and mention of the grant of the patent: **10.10.2018**
(21) Application number: 08781919.9
(22) Date of filing: 16.07.2008
(51) Int. Cl.: A61L 29/04, A61L 29/10, A61L 29/16, A61M 25/00, A61M 25/06

(54) **FOLEY CATHETER HAVING STERILE BARRIER**
FOLEY-KATHETER MIT STERILER BARRIERE
CATHÉTER DE FOLEY À BARRIÈRE STÉRILE

(30) Priority: 16.07.2007 US 949894 P; 24.09.2007 US 974644 P
(43) Date of publication of application: 14.04.2010
(73) Proprietor: C.R. Bard, INC., Covington, GA 30014 (US)
(72) Inventor: NISHTALA, Vasu, Convington, GA 30014 (US); CARLEO, Steven, Covington, GA 30014 (US)
(74) Representative: Rees, Kerry
(86) International application number: PCT/US2008/070227
(87) International publication number: WO 2009/012336

(56) References cited:
- WO-A2-2006/036653
- WO-A2-2007/022223
- US-A- 4 623 329
- US-A- 5 295 979
- US-A1- 2005 283 134
- US-A1- 2006 045 899
- US-A1- 2006 163 097
- US-A1- 2007 161 968
- US-B1- 6 217 569

## Description

### Background

### Field of the Invention

The apparatus disclosed herein relates generally to urethral catheters, and more particularly to improved urethral catheters that minimize the introduction of pathogenic organisms.

### Description of Related Art

In the use of indwelling urethral catheters, such as the Foley catheter, the risk of infection can pose serious problems when the catheter has been indwelling for a few days. Clinical studies have shown that the catheter can provide an avenue for the entry of pathogenic organisms. With respect to organisms gaining access through the interior of the catheter, it has become commonplace to provide means for killing organisms that would otherwise multiply in a urine drainage bag operatively connected to the catheter. Additionally, attempts have been made to prevent organisms from entering the urethral passage between the wall of the urethra and the exterior surface of the catheter.

Attempts aimed at providing a barrier to prevent organisms from entering the urethral passage between the urethra and catheter have sometimes resulted in increased irritation and inflamation of tissue, which materially enhances the likelihood of infection attendant the use of an indwelling catheter. As may be appreciated an indwelling catheter, such as a Foley catheter, is merely exemplary, with similar problems attendant with other drainage tubes as well as venous catheters.

Attempts to provide catheters intended to eliminate or minimize infection have yielded catheters employing a microbiocide capable of withstanding the conditions associated with the manufacture of the catheter. Such catheters often achieve a microbiocidal effect by employing microbiocidal agents in the base material that bleed to the surface. Certain of these, when used in urethral catheters, have resulted in the irritation of the wall of the urethra.

Subsequently developed indwelling catheters resorted to a somewhat different approach in an attempt to reduce infection. Since the tubular body portion of most catheters is formed of a hydrophobic natural or synthetic elastomer, such catheters often have their entire surfaces, both interior and exterior, coated with a hydrophilic polymer to enable the absorbtion of aqueous solutions or suspensions of microbiocides, including antibiotics, into the coating.

For many years silver and silver salts have been used as antimicrobial agents. Silver salts, colloids, and complexes have also been used to prevent and to control infection. For example, colloidal metallic silver has been used topically for conjunctivitis, urethritis, and vaginitis. Other metals, such as gold, zinc, copper, and cerium, have also been found to possess antimicrobial properties, both alone and in combination with silver. These and other metals have been shown to provide antimicrobial behavior even in minute quantities, a property referred to as "oligodynamic."

Additionally, silver is known for antimicrobial use with medical devices, such as catheters, cannulae, and stents. One conventional approach for obtaining antimicrobial medical devices is the deposition of metallic silver directly onto the surface of the substrate, for example, by vapor coating, sputter coating, or ion beam coating. However, these noncontact deposition coating techniques suffer many drawbacks, including poor adhesion, lack of coating uniformity and the need for special processing conditions, such as preparation in darkness due to the light sensitivity of some silver salts.

One particular drawback of these coatings is that the processes by which the coatings are formed do not adequately coat hidden or enclosed areas, such as the interior lumen of a catheter or stent. Additionally, these methods produce coatings that are very much like metallic silver in that they do not release silver from the coating and require contact with the coating to provide antimicrobial action. Though high concentrations of silver may be deposited on the substrate, very little free ionic silver is released on exposure to aqueous fluid. As a result, these coatings provide only limited antimicrobial activity. However, because they do not release sufficient silver ions into aqueous fluids, they offer little or no protection from bacteria carried into the body upon insertion of the device and do not inhibit infection in the surrounding tissue.

With many medical devices, it is useful to have a lubricious coating on the device. Lubricious coatings aid device insertion, reduce the trauma to tissue, and reduce the adherence of bacteria. Another drawback to conventional methods which apply silver and other metals directly onto the surface of a medical device for which a lubricious coating is also desired is that a second, lubricious coating must be applied to the device over the antimicrobial coating, adding to manufacturing cost.

Another approach for obtaining antimicrobial medical devices is the incorporation of silver, silver salts and other antimicrobial compounds into the polymeric substrate material from which the article is formed. An oligodynamic metal may be physically incorporated into the polymeric substrate in a variety of ways. For example, a liquid solution of a silver salt may be dipped, sprayed or brushed onto the solid polymer, for example, in pellet form, prior to formation of the polymeric article. Alternatively, a solid form of the silver salt can be mixed with a finely divided or liquefied polymeric resin, which is then molded into the article. Further, the oligodynamic compound can be mixed with monomers of the material prior to polymerization.

In certain cases, it has been found that irritation may be encountered if a microbiocide is applied to substantially the entire surface of a catheter. Likewise, when an antibiotic is impregnated into the surface of a catheter, only those organisms that are rendered dormant or killed by that particular antibiotic would be effected whereby the protective flora would be damaged, with a possibility that other organisms normally subdued by the flora would run rampant and thus the use of an antibiotic impregnated catheter would tend to induce rather than prevent infection.

Moreover, rendering a surface of a catheter hydrophilic can cause other problems. One of the most significant problems in this regard is brought about by the very nature of the coating, its hydrophilicity, which provides a wettable surface. Thus, once such wettable surface is in contact with a physiological fluid such as urine, for example, which has dissolved salts and other solid compounds in its composition, the hydrophilic coating by virtue of uptake of the aqueous moiety of such physiological fluid can provide a nucleus for the accretion of salt, due to a supersaturated condition adjacent the coating as well as accretion of other solid components of the composition. An unfortunate end result is a plugged catheter or a catheter with a sharp accretion of salts and the like on the exterior surface of the catheter.

U.S. Patent No. 4,055,682 proposes a catheter having a silicone body portion rendered hydrophilic by contacting it with N-vinyl pyrrolidone and exposing the catheter to ionizing radiation. U.S. Patent Nos. 3,566,874 and 3,695,921 propose indwelling Foley urethral catheters made of natural or synthetic rubber having an external coating of a hydrophylic acrylate or methacrylate polymer grafted thereto for the stated purpose of reducing irritation and infection, wherein it is indicated that a hydrophilic polymer may be impregnated with an antibiotic or germicide.

U.S. Patent No. 4,515,593 proposes a catheter having a body portion formed of a hydrophobic elastomer and having a predetermined selected portion of the exterior surface, intermediate the ends, coated with a hydrophilic elastomer for reception of a microbiocide along a limited portion at the site of entry of the catheter into the body.

U.S. Patent No. 7,179,849 proposes antimicrobial compositions, methods for the production of these compositions, and use of these compositions with medical devices, such as catheters, and implants. The compositions are said to provide varying release kinetics for the active ions in the compositions due to the different water solubilities of the ions, allowing antimicrobial release profiles to be tailored for a given application and providing for sustained antimicrobial activity over time. More particularly, polymer compositions are proposed that contain colloids comprised of salts of one or more oligodynamic metal, such as silver. The process proposed includes mixing a solution of one or more oligodynamic metal salts with a polymer solution or dispersion and precipitating a colloid of the salts by addition of other salts to the solution which react with some or all of the first metal salts. The compositions can be incorporated into articles or can be employed as a coating on articles such as medical devices. Coatings are proposed that may be placed upon all or part of a surface.

US 2006/163097 A1 relates to a hydrophilic catheter assembly that is adapted for vapour hydration within a catheter package. The catheter assembly within the package includes a catheter tube having an outer surface with a hydrophilic coating, an introducer tip and a collapsible sleeve.

US 2007/0161968 A1 discloses an implantable medical device comprising an inner region and an outer region positioned over at least a portion of the inner region and in contact with a surface of the inner region. The durometer of the inner region is greater than the durometer of the outer region and a pharmacologically active ingredient is present in at least a portion of the inner region or the outer region.

As may be appreciated, in the use of close urinary drainage systems, several routes for bacterial migration into the bladder exist. Use of the afore-mentioned anti-microbial coatings and others on the inside and outside of Foley catheters have been shown to dramatically reduce the incidence of urinary tract infections (UTIs). However, during the placement of such catheters, aseptic techniques must be employed so as not to drag any bacteria into the urethra during insertion. Despite the sterile handling of catheters during insertion, problems arise. Catheters in use today, once placed, are exposed to the ambient environment and any body fluid spill or the like potentially increases the propensity of extra-luminal ascension of bacteria. Moreover, recognizing that the interface between the meatal opening in the patient and the Foley catheter is protected only be the coating on the catheter, further problems can arise.

### Summary

In accordance with the present invention, there is provided a catheter for indwelling introduction into a body opening, comprising: an elongated body having a lumen and an exterior surface, the body being formed of a polymer and being composed of or having an outside surface coated with a first oligodynamic salt; an introducing member being configured to slide along the exterior surface of said elongated body and being composed of or having an outside surface coated with a second oligodynamic salt, said first oligodynamic salt and said second oligodynamic salt having different solubilities in water so that the rates of release of said first oligodynamic salt and second oligodynamic salt are different; and a polymeric sleeve covering a substantial portion of said elongated body and having a first end and a second end, said first end being secured to said introducing member.

In addition, further advantageous embodiments follow from the dependent claims.

These and other features will be apparent from the detailed description taken with reference to the accompanying drawings.

### Brief Description of the Drawings

The above mentioned and other features of the invention will now be described with reference to the drawings of several embodiments of the present securement devices and systems. The illustrated embodiments of the securement devices and systems are intended to illustrate, but not to limit the invention. The drawings contain the following figures:
FIG. 1 is a perspective view of an urethral catheter according to a preferred embodiment of the present invention;
FIG. 2 is a side elevational view of the catheter of FIG. 1 showing the manner of its placement relative to the urinary tract of a male patient;
FIG. 3 is a side elevational view of the catheter of FIG. 1 showing the manner of its placement relative to the urinary tract of a female patient;
FIG. 4 is a side view of a tip of the catheter;
FIG. 5 is a side view of an introducing member;
FIG. 6 is a side view of a sleeve and an elongated flexible body portion of a catheter;
FIG. 7 is a cross sectional view of the catheter of FIG. 5 showing polymeric coatings on the flexible body portion and the introducing member;
FIG. 8 is a side elevation view of a tear strip;
FIG. 9 is an elevation view of a urine storage bag and associated catheter;
FIG. 10 is a view of a water/air pump;
FIG. 11 illustrates another preferred embodiment of the present invention;
FIG. 12A and FIG. 12B are side views of the catheter of FIG. 11 showing an introducer with a cap and without a cap, respectively;
FIG. 13 illustrates an introducer assembly connected to a catheter;
FIG. 14 illustrates a catheterization method;
FIG. 15 illustrates another aspect of a catheterization method;
FIG. 16 illustrates a catheterization apparatus, inserted into a model; and
FIG. 17 is a process of using a catheter to funnel urine and to protect against bacterial migration.

### Detailed Description of Certain Embodiments

Various aspects will now be described with reference to specific embodiments selected for purposes of illustration. It will be appreciated that the scope of the catheters disclosed herein are not limited to the selected forms. Moreover, it is to be noted that the figures provided herein are not drawn to any particular proportion or scale, and that many variations can be made to the illustrated embodiments. Reference is now made to the figures, wherein like numerals are used to designate like parts throughout.

In one embodiment, a urinary catheter tube is covered by a flexible polymeric sleeve, where the sleeve provides a sterile barrier for protection from contamination by preventing migration or movement of bacteria into the patient. The sleeve prevents contaminants from reaching the exterior surface of the tube, such as through accidental touching of the tube or through contaminants from the air landing on the tube.

The flexible sleeve is attached to a slideable introducing member. The slideable introducing member is adjustably positioned adjacent to the patient's urethra, and the introducing member can slide along the catheter tube according to the depth that the catheter is inserted into a patient. Likewise, the flexible sleeve attached to the introducing member can extend fully to cover at least a majority of the catheter, or the sleeve can contract or bunch up when a smaller amount of the catheter needs to be covered. The sleeve typically covers the catheter portion that would be exposed to the environment, which is of different lengths depending on how far the catheter is in the patient's body during initial insertion or during later adjustments. As such, the protective sleeve provides the patient protection from contamination.

In one embodiment, there is a "touchless" system including a urinary catheter tube. The tube can be covered by a flexible polymeric sleeve that is attached to an introducer. The introducer can have an indwelling segment to assist in the placement of the catheter into the urethra. The touchless system may also include a cap, to provide a true close system. This system minimizes the catheter's contact with the environment.

As used herein and in the claims, the terms and phrases set out below have the meanings which follow.

"Metal" or "metals" includes one or more metals whether in the form of substantially pure metals, alloys or compounds such as oxides, nitrides, borides, sulphides, halides or hydrides.

"Oligodynamic metals" are silver, platinum, gold, zinc, copper, cerium, gallium, osmium, palladium, iridium, tin, antimony, bismuth, or mixtures of these metals with same or other metals

"Noble metals" are silver, gold, platinum and palladium, or mixtures of such metals with same or other metals.

"Antimicrobial activity" means that atoms, ions, molecules or clusters of the antimicrobial or noble metal are released into the solution which the coating contacts in concentration sufficient to inhibit microbial growth on and in the vicinity of the coating. The most common methods of measuring an antimicrobial effect are a zone of inhibition test (which indicates an inhibitory effect, whether microbiostatic or microbicidal) or a logarithmic reduction test (which indicates a microbiocidal effect). In a zone of inhibition test (ZOI) the material to be tested is placed on a bacterial lawn (or a lawn of other microbial species) and incubated. A relatively small or no ZOI (less than 1 mm) indicates a non-useful antimicrobial effect, while a larger ZOI (greater than 5 mm) indicates a highly useful antimicrobial effect. The ZOI is generally reported as a corrected zone of inhibition (CZOI), wherein the size of the test sample is subtracted from the zone. A logarithmic reduction test in viable bacteria is a quantitative measure of the efficacy of an antibacterial treatment; for example, a 5 log reduction means a reduction in the number of microorganisms by 100,000-fold (e.g., if a product contained 100,000 pertinent microorganisms, a 5 log reduction would reduce the number of pertinent microorganisms to 1). Generally, a 3 log reduction represents a bactericidal effect. The logarithmic reduction test involves combining an inoculum of bacteria or other microbial species with the test treatment, incubating the inoculum with the test treatment, recovering the bacteria or other microbial species, and enumerating the bacteria or other microbial species using serial dilutions.

"Anti-inflammatory effect" means a reduction in one or more of the symptoms of erythema (redness), edema (swelling), pain and pruritus which are characteristic of inflammatory conditions.

"Biocompatible" means generating no significant undesirable host response for the intended utility. Biocompatible materials are non-toxic for the intended utility. Thus, for human utility, biocompatible is most preferably non-toxic to humans or human tissues.

"Lubricous polymers" are polymers which become lubricious on wetting with water or a water or alcohol-based electrolyte. Most lubricious polymers are hydrophilic, but some hydrophobic polymers may also function as lubricious polymers if they have a sufficient degree of lubricity on wetting.

"Hydrophilic" means that water droplets do not readily form beads on the surface of such hydrophilic material, but instead, the water droplets tend to assume a contact angle of less than 45 degrees and readily spread on its surface. The term "hydrophilic polymer" is meant to include polymers which are hydrophilic on wetting and which also produce a lubricity in that wetted state. "Hydrophilic polymer" is also meant to include "water swellable" polymers, wherein "water swellable" means a substantially hydrophilic polymer which, even though not soluble in water, absorbs sufficient water to render it lubricious in the hydrated state. While these definitions all refer to water as an agent for hydration, it should be understood to include other water or alcohol-based electrolytes including bodily fluids, which are capable of hydrating or swelling the polymer.

"Hydrophobic" means that water droplets readily form beads on the surface of such hydrophobic material.

"Solvent" is the term used herein to describe the liquid medium used to solubilize, disperse or suspend the components of the coatings disclosed herein prior to applying the coating to the substrate. As used herein, the term does not imply that the components of the coatings are completely dissolved in the solvent or is otherwise effective in promoting some swelling of the polymer.

A catheter can be used for multiple purposes. For example, a catheter can be used as a urinary catheter, which is a device that enables a medical patient to go to the bathroom without having to leave the patient's bed. This can be useful for males and females with medical conditions. For example, patients with urinary incontinence, a condition otherwise known as an involuntary leakage of urine, may have cost benefits to a short term usage of the catheter. Another example is a patient undergoing a major surgery, or a surgery that limits the patient's movement, such treatment in an intensive care unit (ICU), which requires accurate monitoring of medical device inputs and outputs. In this example, the patient may benefit by using the catheter for a longer period of time.

In urinary catheterization, a plastic tube known as a urinary catheter (such as a Foley catheter) is inserted into a patient's bladder via their urethra. A balloon located at the end of the catheter inside the patient can be inflated with sterile water to prevent the catheter from slipping out once it has reached the bladder. In this manner, the patient's urine can be transferred from the bladder to an external urine storage bag. In another embodiment, the Foley catheter is used to inject liquid into a bladder. Catheterization is usually performed by a clinician, often a nurse, although self-catheterization is possible as well.

One concern during urinary catheterization is urinary tract infections. This can be caused by pathogenic organisms that come in contact with the exterior of the catheter, which may be transferred into the patient as the device is inserted or adjusted. In one embodiment, the catheter device includes a slideable and flexible sleeve and introducing member for covering the tube and preventing pathogenic organisms from coming in contact with the exterior of the catheter.

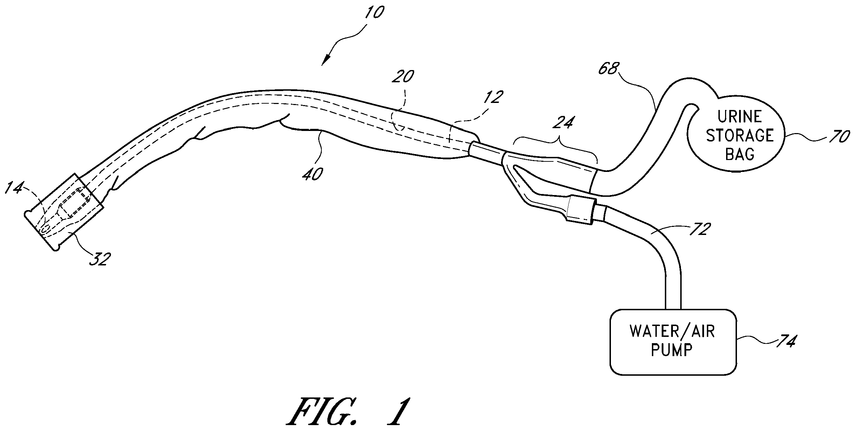

To assist in the description of the components of embodiments of the anchoring system, the following coordinate terms are used. A "longitudinal axis" is generally parallel to a section of the catheter. In Figure 1, the longitudinal axis is generally parallel to a elongated flexible body portion 12. As used herein, "the longitudinal direction" refers to a direction substantially parallel to the longitudinal axis. The term "distal" is used in reference to the end of catheter 10 near the patient's body. The term "proximal" is used in reference to the end of catheter 10 near the funnel 24.

Referring to FIG. 1, there is shown a catheter 10, such as a Foley urethral catheter, for introduction into a body opening. The catheter 10 may be inserted for a short or long period of time. The catheter 10 includes an elongated flexible body portion 12 formed of a polymer, which terminates in a tip portion indicated generally at 14. The elongated flexible body portion 12 has a first end and a second end and at least one inner lumen 20. The inner lumen 20 may be employed for drainage. As shown, the elongated flexible body portion 12 also has an exterior surface. An introducing member 32 is affixed to a sleeve 40 on a distal end (near patient) of the catheter 10.

The catheter 10 further includes a funnel 24 on its proximal end which splits into a inflation lumen and a drainage lumen 20. The inflation lumen can connect to a water/air pump 74 through a tube 72. The water/air pump 74 is employed to flow water or air into the inflation lumen.

On its proximal end, the drainage lumen of the funnel 24 passes urine through tube 68 to a urine storage bag 70. The funnel 24 preferably connects to the urine storage bag 70 and the water/air pump 74. While the funnel 24 may be connected to the urine storage bag 70 for an extended period of time, it may only need to be connected to the water/air pump 74 for a long enough period of time to inflate the distal end of the catheter 10 in the bladder.

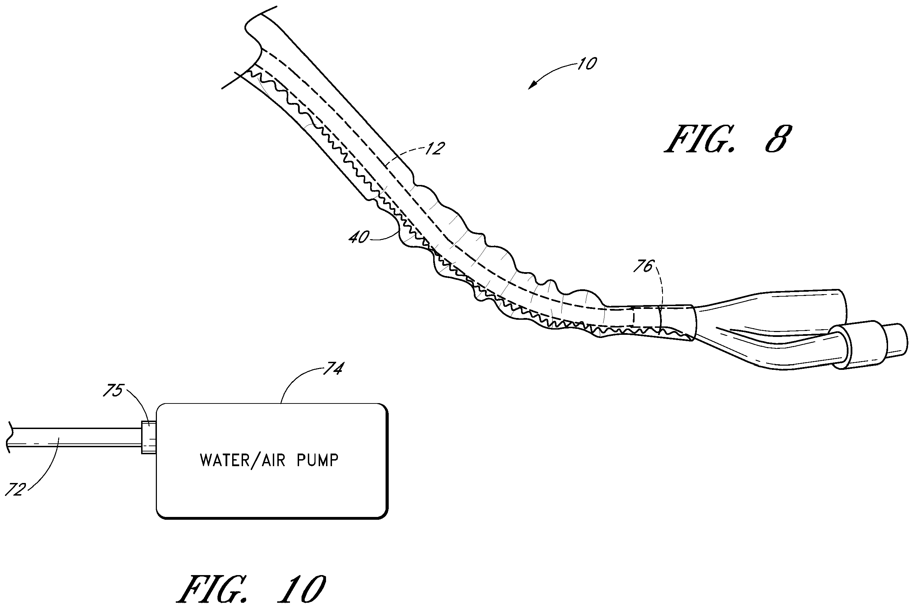

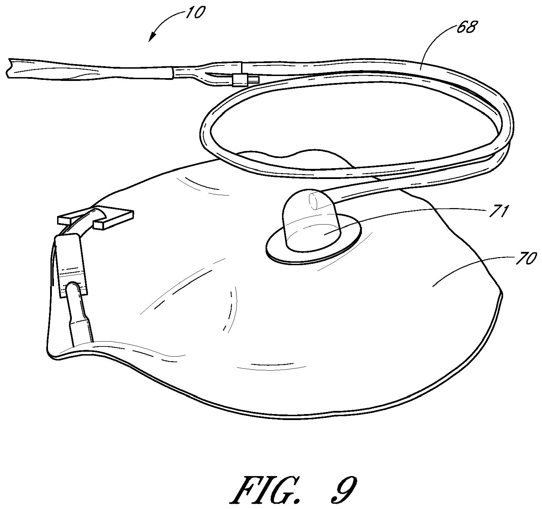

The first opening of the funnel 24 exits urine into a urine storage bag shown in FIG. 9. The second opening connector inserts fluids into the retaining bag or balloon from a pump shown in FIG. 10.

The sleeve 40 may include a tear strip as is illustrated in FIG. 8. The tear strip may be used to remove the sleeve 40 from the catheter 10 after the catheter 10 is placed. In another embodiment, the tear strip is positioned to tear off an excess length of the sleeve 40.

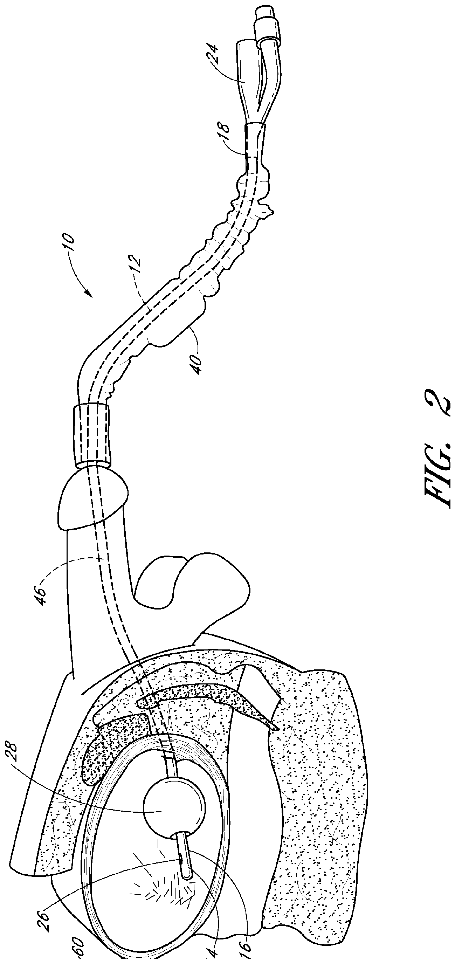

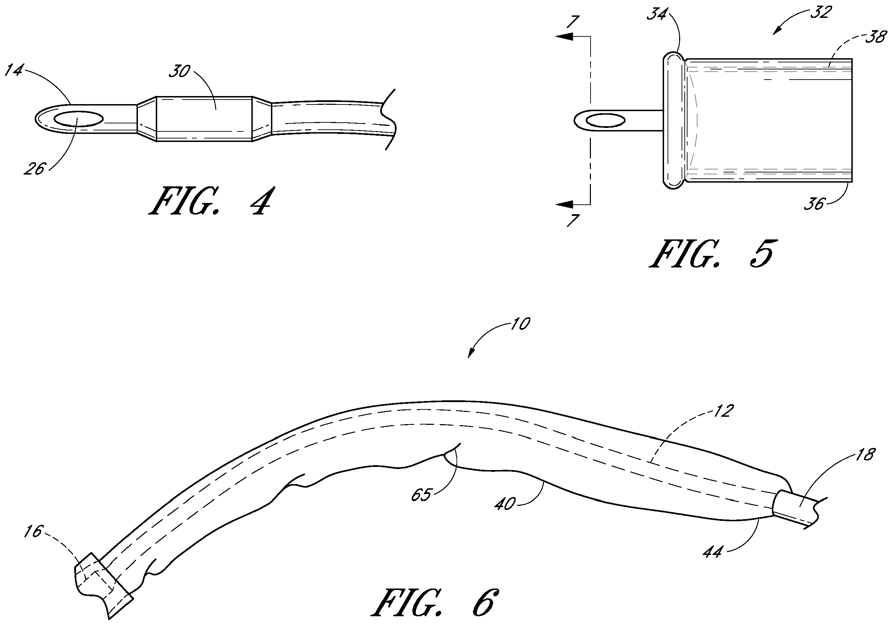

FIG. 2 is a side elevational view of the catheter of FIG. 1 showing the manner of its placement relative to the urinary tract of a male patient. A drainage lumen on the second end 18 of the elongated flexible body portion 12 can connect the funnel 24 on the proximal end, with a drainage port 26 inside the patient, for transferring urine from the patient's bladder 60 to the urine storage bag. The flexible body portion 12 of the catheter 10 extends from the bladder 60, through the meatus 46 opening in the patient, through the introducing member and sleeve 40 and finally to the funnel 24. At the distal end, an inflatable retaining bag or balloon 28 encompasses the elongated flexible body portion 12, at a point inwardly of drainage port 26. The balloon 28 is sealed or otherwise connected thereto in conventional fashion. A longitudinally extending inflation lumen (shown in FIG. 4) terminates in an inflation port. The inflation port communicates with the interior of the balloon 28 and with a valve end portion or arm for the introduction of water or possibly air. The water inflated the balloon 28 so as to retain the tip 14 on the first end 16 of the body 12 of the catheter 10 in the bladder 60.

The elongated flexible body portion 12 may be formed from a relatively wide variety of relatively flexible polymers or elastomers, such as silicone rubber, which is hydrophobic and generally inert with respect to physiological fluids it contacts and biocompatible, as well.

The flexible polymeric sleeve 40 includes a first end and a second end. The first end of the flexible polymeric sleeve 40 is affixed to the flexible introducing member 32. As shown in FIG. 2, the flexible polymeric sleeve 40 is of a length sufficient to cover at least a substantial portion of said elongated flexible body portion 12. The flexible polymeric sleeve 40 may be formed of a sheet forming polymer such as polyvinylidene chloride, polyethylene, polypropylene, polyester or copolymers and terpolymers thereof. In certain forms, the flexible polymeric sleeve 40 is formed of polyvinylidene chloride, low density polyethylene or linear low density polyethylene.

In one form, the first end of flexible polymeric sleeve 40 is coaxially affixed within the flexible introducing member 32 from about the first end of the introducing member to about the second end of the introducing member 32.

The elongated flexible body portion 12 of the catheter 10 and flexible introducing member 32 may be coated by one or more surfaces with a polymeric coating such as set forth in U.S. Patent No. 7,179,849.

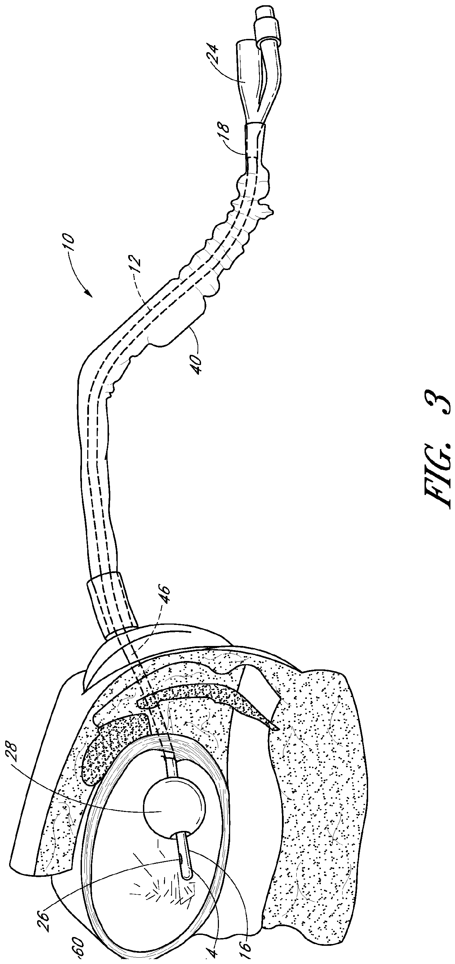

FIG. 3 is a side elevational view of the catheter of FIG. 1 showing the manner of its placement relative to the urinary tract of a female patient. As shown in this figure, a Foley catheter can be used with female patients. In this embodiment, the catheter is used to remove urine. Like FIG. 2, the drainage port 26 inside the patient transfers urine from the patient's bladder 60 to the urine storage bag.

FIG. 4 is a side view of the tip 14 of the catheter. The tip 14 is typically smaller to ease insertion into the meatus opening of the patient. The tip is shown as located on the patient's end of the tube. In the tip 14, a drainage port 26 is shown that allows fluid to enter and exit from the tip. Near the tip 14 is an inflation port 30 which supplies a retaining bag or balloon with air or fluid.

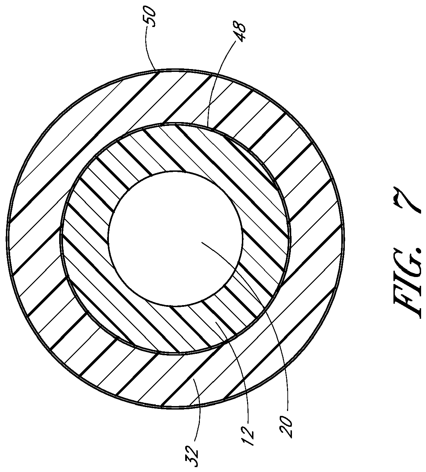

FIG. 5 is a side view of the slideable introducing member 32. The introducing member 32 includes a first end 34 and a second end 36 and a longitudinal bore 38. The longitudinal bore slideably positions the flexible introducing member 32 over and along the exterior surface of the elongated flexible body portion 12. The introducing member 32 can be attached to the first end of the sleeve, so that the sleeve can adjustably extend along the proper length of the catheter. Flexible introducing member 32 may be formed form a wide variety of hydrophobic and hydrophilic polymeric materials. Suitable polymers include, but are not limited to, polyethylene, polypropylene, polyester or copolymers and terpolymers thereof. A polymeric coating on the tube may be applied to minimize introduction of organisms and reduce irritation. Likewise, a coating on the introducing member 32 may be applied. Coatings are further discussed in FIG. 7.

FIG. 6 is a side view of the sleeve 40 and elongated flexible body portion 12 of a catheter 10. The second end 44 of the sleeve 40 is shown attached near the second end 18 of the elongated flexible body 12. In this embodiment, the first end 16 of the sleeve 40 is shown terminating near the first end of the body 12. However, in use, the first end of the body 12 is inserted inside the bladder, and therefore the sleeve 40 can then cover less than the entire length of the tube.

The sleeve 40 provides a barrier to inhibit microorganisms from landing on the body 12 or accidental contact of the body 12 during insertion or use. In the illustrated embodiment, the sleeve 40 is attached to the slideable introducing member 32. The sleeve 40 can slide along the catheter. As such, the sleeve 40 has flexibility 65 to bunch up or accordion as the introducing member 32 slides away from the patient. The flexibility of the sleeve 40 allows the sleeve 40 to increase its length as the introducing member 32 slides adjacent to the patient.

As shown in FIG. 7, the polymeric coating 48 is grafted to the exterior surface of elongated flexible body portion 12. Likewise, polymeric coating 50 is shown as grafted to an exterior surface of the flexible introducing member 32.

The polymeric coating may be applied along at least a portion of the catheter. In one embodiment, the polymeric coating 48 may be applied along the entire length of the elongated flexible body portion 12. Alternatively, coating 48 may be applied along only so much of elongated flexible body portion 12 so as to be positioned and be of a sufficient extent for use in conjunction with or inside of a male or female patient. In this regard, and with specific reference to FIGS. 2-3, the catheter 10 is shown operatively positioned within the urethral tract of a male or female patient, with that portion thereof provided with an exterior coating 48 generally contiguous with the meatus, which is a body opening such as an inner wall of a uretha, so that the polymeric coating 48 at least straddles both ends of the meatus 46 of the patient. With this arrangement, at least the inner wall of the uretha is subjected to the microbiocide.

The polymeric coating may be added to the catheter at various times. In one embodiment, the polymeric coating 48 is provided at the time of manufacture of the catheter 10 and the coated catheter 10 packaged under aseptic conditions or packaged and sterilized by suitable means, for subsequent use. In such instance it will be appreciated that by using an accepted packaging technique the catheter would be removed from its sterile package and the tip portion 14 passed upwardly through the urethra for placement of the catheter 10 as illustrated in FIGS. 2-3.

Polymeric coating may be applied along different portions of the catheter. In one embodiment, polymeric coating 48 is restricted to the exterior of the body portion 12 and does not include a comparable coating on the inner wall defining the drainage lumen 20, as best seen in FIG. 7. In this way, encrustation and/or plugging of the drainage lumen may be obviated. In the form wherein the longitudinal extent of the application of the microbiocide to the polymeric coating 48 is minimized, the reduced contact of microbiocide with body tissue can serve to greatly minimize the irritation of body tissue, it being appreciated, of course, that to some degree or another virtually all microbiocides comprise tissue irritants.

FIG. 8 is an elevation view of a tear strip 76 extending between the distal and proximal ends of the sleeve 40. The tear strip 76 can be used to remove the entire sleeve 40 from the body 12, or just an excess portion of the sleeve 40 following the placement of the catheter 10. As shown in FIG. 8, the sleeve 40 is bunched up over the body portion 12 of the tube. The entire sleeve 40 or an excess length of the sleeve 40 can be removed by cutting along the tear strip 76.

FIG. 9 is an elevation view of a urine storage bag 70. The urine storage bag 70 has an opening 71 in the bag 70. The opening 71 is connected through a tube 68 to the funnel on the proximal end of the catheter 10. The urine storage bag 70 can be disposable for one time use or designed to be emptied multiple times.

FIG. 10 is a side view of a water/air pump 74. The pump 74 can pump water through an opening 75 and a tube 72 into the funnel of the catheter, and ultimately into a retaining bag or balloon located in the patient's bladder. The balloon retains the tip of the catheter in the bladder, and maintains the drainage port in the proper position to allow urine to pass through.

As indicated below, in the use of a closed urinary drainage system, there are several routes for bacterial migration into the bladder. Use of the anti-microbial coatings disclosed herein on the inside and outside of the Foley catheters can dramatically reduce incidence of UTIs. During placement of catheters, aseptic techniques are employed so as not to drag any bacteria into the urethra during insertion. The use of the catheters disclosed herein having a protective sterile sleeve over the catheter can serve to minimize contamination issues, there by enhancing nursing efficiency. Unlike Foley catheters of prior designs, wherein the interface between the meatal opening in the patient and the Foley catheter is protected only by the coating on the catheter, the catheters disclosed herein provide an integrated solution.

Referring again to FIGS 1-10, flexible introducer 32 is designed to have a different anti-microbial activity profile than the polymeric coating 48 of elongated flexible body portion 12, to provide a concentrated quick kill type effect, as described hereinabove, that could either be left in place; essentially shutting off any routes of entry for bacteria or removed after placement. As described hereinabove, flexible introducer 32 is coaxially attached to a flexible polymeric sleeve 40 that fits over elongated flexible body portion 12 of the catheter 10 and is attached near the funnel 24. As may be appreciated by those skilled in the art, with this configuration, the outside can be handled and the inside is sterile as the catheter 10 is placed and during its indwelling time in the patient. When the elongated flexible body portion 12 of the catheter 10 advances into the urethra, the flexible sleeve 40 accordions, as shown in more detail in FIGS. 2-3. Optionally, a tear strip (FIG. 8) can be provided and stripped and removed after placement.

Also provided is a catheter for use in a method of minimizing likelihood of infection due to an indwelling catheter. The method includes the steps of providing a catheter, the catheter including an elongated flexible body portion formed of a polymer, the elongated flexible body portion having a first end and a second end, at least one inner lumen and an exterior surface, a flexible introducing member, the flexible introducing member having a first end and a second end. about the exterior surface of the elongated flexible body portion, and a flexible polymeric sleeve, the flexible polymeric sleeve having a first end and a second end, the first end of the flexible polymeric sleeve affixed to the second end of the flexible introducing member. In one embodiment, the flexible polymeric sleeve is a length sufficient to cover at least a substantial portion of the elongated flexible body portion and placing the catheter into the body of a patient by grasping the flexible introducing member without contacting the elongated flexible body portion, whereby entry of pathogenic organisms through the body opening is minimized.

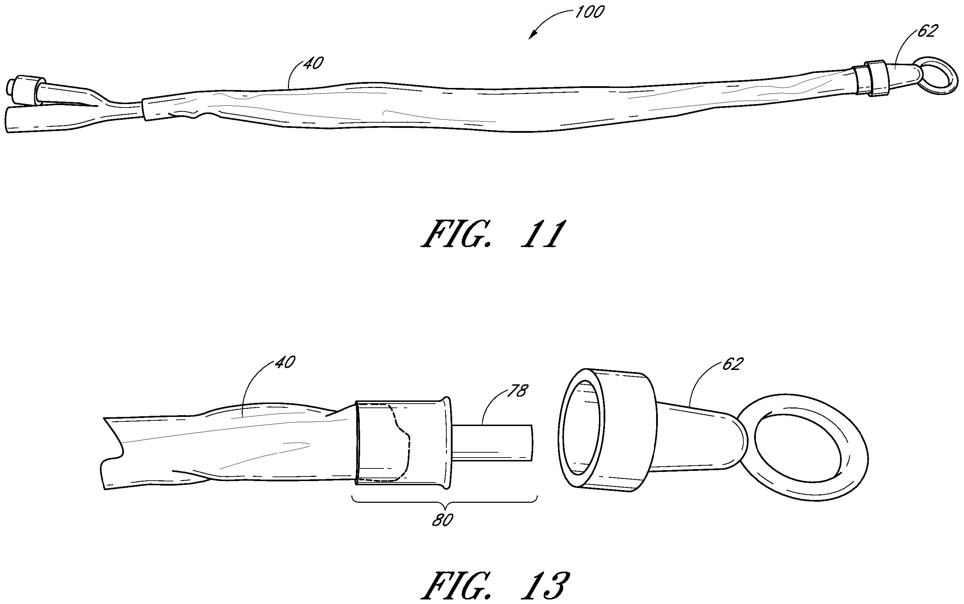

FIGS. 11-16 illustrate another "touchless" catheterization system in accordance with a preferred embodiment of the present invention. The catheter 100 illustrated in Figure 11 is similar to the catheter 10 illustrated in Figure 1 except that the introducer member 32 is replaced with an introducer 80 / indwelling segment 78. One of the benefits of a touchless system using catheter 100 is minimization of contact between the Foley catheter and the surrounding environment while preventing, or at least minimizing, extra-luminal migration of bacteria along the catheter shaft. This touchless system can be used for both male and female patients.

As may be appreciated, the embodiments of the catheters disclosed herein provide an added layer of protection for infection control in the product life cycle of Foley catheters and drainage systems. They potentially eliminate the incidence of accidental touch contamination and offer a barrier for bacteria moving from outside of the catheter into the bladder.

The touchless catheterization system illustrated in FIG. 11 comprises a sleeve 40, an introducer 80, and a catheter 100. The system may further include a cap 62. At least a portion of the introducer 80 is placed within the urethra. In the illustrated embodiment the portion is an indwelling segment 78. The segment 78 can be made from a soft and flexible material. The sleeve 40 illustrated in FIG. 11 envelopes the entire shaft of the catheter 100, including the tip. The cap 62 can act as a reservoir for an agent, such as a lubricant or an antiseptic agent. The cap 62 provides for a true closed system.

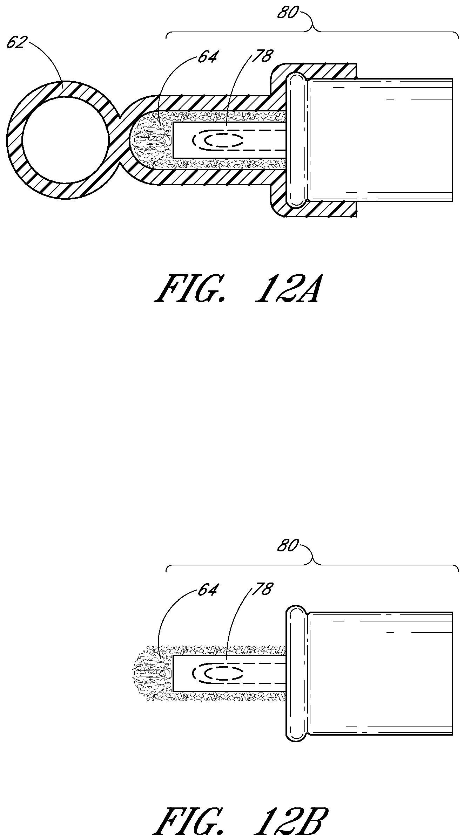

FIG. 12A and FIG. 12B are a side views of the introducer 80 with and without a removable cap 62. In FIG. 12A, the introducer 80 is shown with a cap 62. The cap 62 provides additional protection from bacteria when the catheter is not in use. As shown, the cap 62 attaches to the introducer 80 which includes an indwelling segment 78. In another embodiment, the cap could attach to an end of the elongated flexible body portion 12. An opening inside the cap 62 and near the introducer 80 can be used to store a lubricant or antiseptic agent 64.

In FIG. 12B, the introducer 80 is shown without a cap. Agent 64 is shown around the elongated flexible body of the tube. The agent 64 provides an additional means of minimizing infection. Alternatively, the lubricant 64 can minimize the irritation from inserting the catheter into the urethra.

FIG. 13 illustrates the introducer 80 / indwelling segment 78. The introducer 80 is located on the distal end (i.e., the patient end) of the sleeve 40 and is comprised of a semi-rigid polymer. The indwelling segment 78 is an extension of the introducer 80.

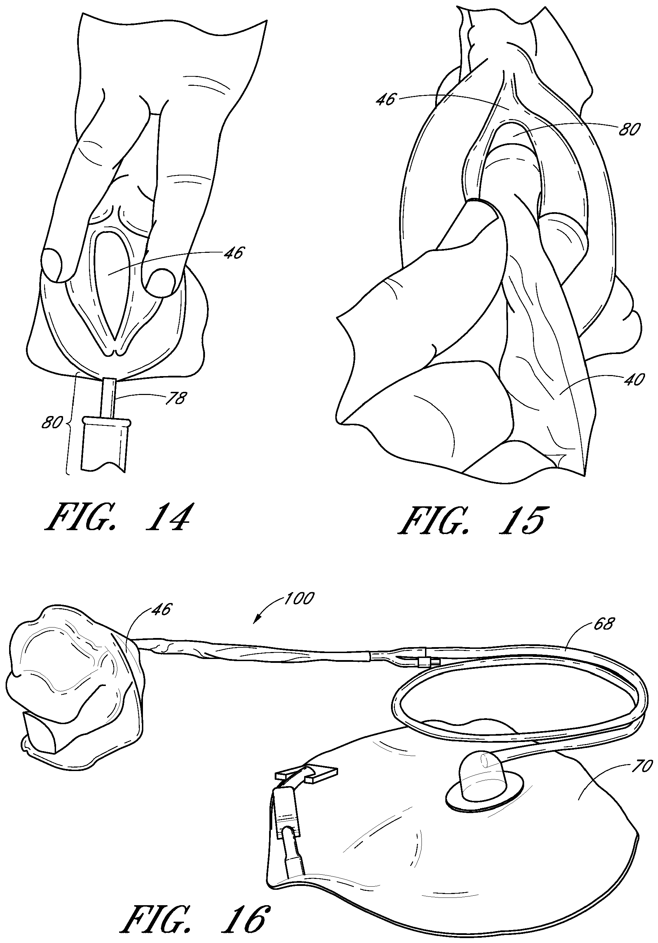

While the patient is being prepared for catheterization, the "no touch," or "touchless," system minimizes the catheter's contact with the environment. In FIGS. 12-16, the process for insertion of the catheter 100 is illustrated. First, the cap 62 can be removed from the system as shown in FIG. 13. As a result, the lubricant and/or antiseptic agent is left behind on the indwelling segment 78 of the introducer 80 as shown in FIG. 12B. Next, the indwelling segment 78 is placed into the meatus 46 of the urethra as shown in FIGS. 14 and 15. During the insertion process, the indwelling segment 78 allows the nurse to easily gain access to the urethra and provide support during the insertion process. The lubricant and/or antiseptic agent 64 can spread across the catheter shaft as it is advanced through the introducer 80. While the catheter 100 is in use, the sleeve 40 prevents direct contact between the catheter 100 and is surrounding environment (e.g., contaminants within the room, bodily fluids). In FIG. 16, the catheter 100 is shown inserted into the meatus 46 of a model of a patient. Further, the drainage lumen tube 69 is shown for exiting urine to the urine storage bag 70.

When the catheter is being removed, the sleeve provides a barrier between the nurse and the catheter. When fully removed, the cap can be placed on the introducer to prevent spillage of residual urine.

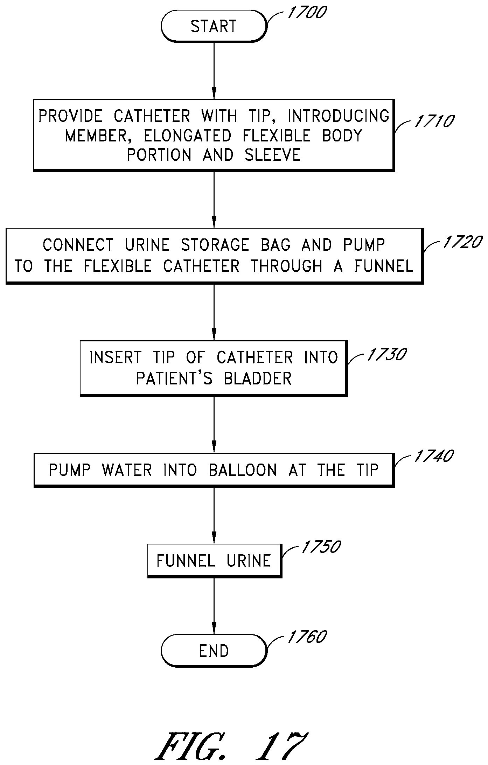

FIG. 17 is a process of using the catheter. It will be appreciated by the skilled practitioner that the illustrated process can be modified in a variety of ways. For example, various portions of the illustrated process can be combined, can be rearranged in an alternate sequence, can be removed, or the like.

The process starts at step 1700. At step 1710, a catheter is provided containing a tip, introducing member, elongated flexible body portion, and sleeve. Subsequently, at step 1720, a patient or medical provider connects a urine storage bag and a pump to the catheter through a funnel. Next, at step 1730 the tip of the catheter is inserted into the patient's bladder. At step 1740, water is pumped into the balloon located at the tip of the catheter and inside the bladder to maintain the catheter in the bladder and to maintain the drainage port at the appropriate location within the bladder. Subsequently, the patient's urine is funneled through the catheter and into a urine storage bag at step 1750. The process ends at step 1760.

The following detailed description provides examples and details related to urinary catheterization. Specifically, details related to the polymeric coatings 48 and 50 are provided, including their concentration and time release characteristics.

The polymeric coatings 48, 50 may comprise many agents. Also, polymeric coating 48 may be the same or different than polymeric coating 50. For example, the antimicrobial compositions that comprise polymeric coatings 48 and 50 may include a polymer and a colloid comprised of the salts of one or more oligodynamic agents. The term "oligodynamic agent" refers to any compound that can provide antimicrobial activity, even when present in small quantities.

Any polymer may be employed, including hydrophilic polymers, hydrophobic polymers, and mixtures of these two types of polymers. The use of hydrophilic polymers is advantageous because such polymers have additional benefits. These benefits include increased lubricity for patient comfort, increased absorption of aqueous fluids from the body which aids in the release of oligodynamic ions from the composition, inhibition of bacterial attachment, and improved solubility for some metal salts. Useful hydrophilic polymers are those that are soluble in water or in organic solvents containing water. The ability to add water to the polymer composition without precipitating the polymer facilitates the addition of water-soluble salts directly to the coating composition. Water facilitates the formation of salt colloids within the polymer composition. For this reason, the polymer solution may contain from 1 to 50% water by weight, more preferably from 5 to 30% water by weight.

The use of water is not limiting, as salt colloids can also be formed using alcohols, organic solvents, or both that contain little or no water. The use of alcohols and organic solvents, containing from 0 to 1% water may be used when hydrophobic polymers are employed.

Examples of polymers which may be used to form the compositions include, but are not limited to, polyurethanes, including polyether polyurethanes, polyester polyurethanes, polyurethaneureas, and their copolymers; polyvinylpyrrolidones; polyvinyl alcohols; polyethylene glycols and their copolymers; polypropylene glycols and their copolymers; polyoxyethylenes and their copolymers; polyacrylic acid; polyacrylamide; carboxymethyl cellulose; glycoproteins; proteoglycans; glycosaminoglycans; lipoproteins; liposaccharides; cellulose and its derivatives; dextrans and other polysaccharides; starches; guar; xantham and other gums and thickeners; collagen; gelatins; other naturally occurring polymers; polytetrafluoroethylene; polyvinyl chloride (PVC); polyvinylacetate; poly(ethylene terephthalate); silicone; polyesters; polyamides; polyureas; styrene-block copolymers; polymethyl methacrylate; acrylic-butadiene-styrene copolymers; polyethylene: polystyrene; polypropylene; natural and synthetic rubbers; acrylonitrile rubber; and mixtures and copolymers of any of the above. The selection of a polymer depends upon the substrate to be coated. In one form, the polymer is a polyurethane or polyurethane copolymer, such as polyether polyurethaneurea. In another form, hydrophobic polymers that are chemically similar or identical to the substrate are used alone or in combination with hydrophilic polymers to form coatings that enhance adhesion of the coating to the substrate.

The colloid comprises one or more oligodynamic salts. The oligodynamic metal cations come from the salts referred to as salt A. In another form, the oligodynamic salts comprise one or more salts of oligodynamic metals. The salts may be different salts of the same oligodynamic metal or may be salts of different oligodynamic metals. Oligodynamic metals useful herein include, but are not limited to, silver, platinum, gold, zinc, copper, cerium, gallium, osmium, and the like. In yet another form, the oligodynamic metal is silver.

Salts of other metals may be employed to form the colloid. In the discussion below, these salts are referred to as salt B. These salts contain cationic ions that include, but are not limited to, calcium, sodium, lithium, aluminum, magnesium, potassium, manganese, and the like, and may also include oligodynamic metal cations such as copper, zinc, and the like. These salts contain anions that include, but are not limited to, acetates, acetylsalicylates, ascorbates, benzoates, bitartrates, bromides, carbonates, chlorides, citrates, folates, carbonates, deoxycholates, gluconates, iodates, iodides, lactates, laurates, oxalates, palmitates, para-aminobenzoates, para-aminosalicylates, perborates, phenosulfonates, phosphates, picrates, propionates, salicylates, stearates, succinates, sulfadiazines, sulfates, sulfides, sulfonates, tartrates, thiocyanates, thioglycolates, thiosulfates, and the like, as well as silver proteins and silver ethylenediaminetetraacetic acid. Oxides that may also serve as Salt B, include, but are not limited to oxides of calcium, sodium, lithium, aluminum, magnesium, potassium, manganese, and the like, and may also include oligodynamic metal cations such as copper, zinc, and the like.

The compositions can contain auxiliary components. Examples of such auxiliary components include, but are not limited to, viscosity and flow control agents, antioxidants, conventional pigments, air release agents or defoamers, and discolorants. The composition may also contain conventional dyes and pigments to impart color or radiopacity or to enhance the aesthetic appearance of the compositions. The compositions can also contain additional lubricating agents and other additives that enhance patient comfort and tissue health.

While not wishing to be bound by the following mechanism, it is believed that many of the advantageous properties of some forms disclosed herein result from the differences in the solubility of the different metal salts present in the colloid. These differing solubilities of the metal salts in the colloid provide varying release kinetics for the active oligodynamic metal(s). For example, with a medical device composed of, or coated with, the compositions disclosed herein, those salts that have high water solubility will be released from the coating rather quickly, providing a high initial dose of antimicrobial activity to kill bacteria introduced upon insertion of the device in the patient. This initial dose is sometimes referred to as "quick kill," and this antimicrobial activity is identified by the ability of a coated device or composition to create zones of no bacterial growth around the device or composition when it is placed in a bacterial culture. This test is known as a "zone of inhibition" assay. Those salts having lower water solubilities will be released more slowly from the composition, resulting in a sustained or extended antimicrobial activity over time.

Selection of salts having varying degrees of solubility in the composition allows tailoring of the composition to the specific application of the article comprising the composition. In one form, compositions disclosed herein are tailored to kill bacteria introduced during the insertion of catheter 10 or catheter 100, both on the surface 22 of the flexible body portion 12, and on the flexible introducing member 32 or the introducer 80, and in the surrounding fluid and tissue, by the quick release of antimicrobial metal salts, followed by prolonged inhibition of bacterial migration and growth by the slower release of less soluble antimicrobial metal salts over an extended period of time. The compositions may also contain silver salts with a very low solubility, thus reducing the release of silver into the fluid surrounding the article in order to reduce tissue exposure to silver ions while maintaining inhibition of microbial adherence on the surface of the coated article. The ability to tailor the release of the oligodynamic agent is advantageous over conventional antimicrobial compositions, as it provides for both immediate and sustained antimicrobial activity.

The composition may contain any amount of one or more oligodynamic metal salts, oxides, or combination of salts and oxides. In some forms, the composition contains between about 40% and about 50% (based on weight of total solids in the composition) of the one or more oligodynamic metal salts, oxides, or combination of salts and oxides, or between about 30% and about 40%, or between about 20% and about 30%, or between about 15% and about 25%, or between about 10% and about 20%, or between about 5% and about 15%, or between about 3% and about 8%, or between about 4% and about 6% (based on weight of total solids in the composition) of the one or more oligodynamic metal salts, oxides, or combination of salts and oxides. In some forms, the composition contains about 5% (based on weight of total solids in the composition) of the one or more oligodynamic metal salts, oxides, or combination of salts and oxides, or greater than zero and up to about 5%, or greater than zero and up to about 2%, or between about 3% and about 4% (based on weight of total solids in the composition) of the one or more oligodynamic metal salts, oxides, or combination of salts and oxides. In some forms, the composition contains about 2.5% (based on weight of total solids in the composition) of the one or more oligodynamic metal salts, oxides, or combination of salts and oxides, or about 1% (based on weight of total solids in the composition) of the one or more oligodynamic metal salts, oxides, or combination of salts and oxides.

In some forms, the coated catheters will reduce adherence of one or more bacteria, fungi, or other microbes as compared to uncoated catheters. In one form, the coating results in an in vitro decrease in microbial adherence of 5 to 95%. In certain forms, the coating results in a decrease in microbial adherence of at least about 30%, or at least about 50%, or at least about 75%, or at least about 90%, or at least about 95%. As used herein, reduction of microbial adherence is determined using the procedures set forth in Example 18 of U.S. Patent No. 7,179,849

In one form, the coated catheters have antimicrobial effects upon surrounding tissues and fluids, as can be demonstrated through zone of inhibition testing on one or more species or strains of bacteria, fungi, or other microorganisms. Examples of antimicrobial effects include, but are not limited to, inhibition of growth, killing, and any other deleterious effect on microbes. In another form, no zone of inhibition is created. In still other forms, limited zones of inhibition are created. Certain forms also exist in which zones of inhibition are created for some strains in a species but not others, or for some species but not others. Other forms exist in which zones of inhibition differ between microbes. As used herein, zones of inhibition are determined using the procedures set forth in Example 19 of U.S. Patent No. 7,179,849.

A catheter may be coated with a composition comprising colloidal silver chloride. The resulting catheter reduces or eliminates adherence of microbes on the surface of the catheter but releases silver to surrounding tissues at such a slow rate due to the low solubility of silver chloride that the catheter does not produce zones in the zone of inhibition assay.

Oligodynamic metals may be released at various times. By tailoring the release profile of the oligodynamic metals, it is possible to develop a catheter having any combination of antimicrobial effects on the surface and surrounding tissues and fluids. Thus, any of the above combinations of effects are achieved. For example, in some forms microbial adherence of a specific species or strain of organisms is reduced while these forms produce little or no zone of inhibition for the same species or strain. Forms also exist in which both zone of inhibition and microbial adherence differ between organisms.

Different quantities of oligodynamic metals may be released. For example, anywhere from 5 to 100% of the oligodynamic metals in the compositions can be released in the first 24 hours. A variety of release profiles from a single catheter are therefore achieved. In some forms, between 75% and 100% of the oligodynamic metal in the coating is released in the first 24 hours. In other forms, between 50% and 75% of the oligodynamic metal in the coating is released in the first 24 hours, or between 25% and 50%, or between 0% and 25% of the oligodynamic metal in the coating is released in the first 24 hours. In other embodiments, about 75% of the oligodynamic metal is released in the first 24 hours, or about 40% of the oligodynamic metal is released in the first 24 hours. Other forms involve releases over a longer period of time. In one such form, about 38% is released the first day and about 80% released within 21 days. As used herein, release is determined using the procedures set forth in the elution tests in Example 20 of U.S. Patent No. 7,179,849.

Another advantage of the polymeric coating compositions is the wet coefficients of friction (COF) achievable. Coating compositions may be manipulated so that highly lubricious coatings are made or hydrophilic coatings with little lubricity are made. Forms exist with intermediary COF values ranging between about 0.100 and about 0.0300 to reduce the risk of unwanted slippage or movement of a coated catheter after placement in a location in the body, while providing enough hydrophilicity to reduce tissue irritation and inflammation. In other forms where a highly lubricious surface is desired, a COF ranging between about 0.040 and about 0.060 (after one hour immersion in water) may be achieved, or between about 0.300 and about 0.400, or between about 0.100 and about 0.200, or between about 0.200 and about 0.300 after one hour immersion is achieved. In another embodiment, a COF ranging between about 0.337 and about 0.373 after one hour immersion is achieved, or between about 0.040 and about 0.060, or between about 0.100 and about 0.300 after one hour immersion is achieved. As used herein, COFs are determined using the procedures set forth in Example 21 of U.S. Patent No. 7,179,849.

Although that example deals with endotracheal tubes, it may be used for any coated surface, such as a catheter.

Another advantage of the compositions used to form the polymeric coatings disclosed herein is that the formation of colloids within the polymer composition produces ultra-fine particles that possess a minimal particle size for the metal salts. This minimal particle size retards settling and agglomeration. The use of colloids in the composition also permits incorporation of higher quantities of antimicrobial metal without the difficulties associated with the suspensions used in the prior art.

By reducing or eliminating the problems associated with conventional antimicrobial polymer compositions, reproducible compositions having specific antimicrobial ion concentration with a specific antimicrobial ion release profiles that can be tailored through the specific salt combinations selected to provide optimum antibiotic activity over an extended period of time may be provided. For example, such compositions can be tailored to release the bulk of their oligodynamic agents within 5 days, or within 14 days, or within 30 days for a device with a longer term use, such as a Foley catheter. Longer and shorter terms are possible.

The tailored concentration and time release delivery will now be further described in terms of a polyurethane composition containing a colloid of specific silver salts. It is to be understood that this is simply an example of one form and that one of skill in the art, based upon the present disclosure, can pick and choose salts having differing solubilities to provide a composition having a suitable release profile for a particular purpose.

In one embodiment, a coating solution is formed from a 4.7% solution of a polyether polyurethane-urea block copolymer available from CardioTech International, Inc. in a mixture of THF/alcohol in a 75/25 ratio by weight. A sufficient quantity of 10% silver nitrate (AgNO₃) solution in water is added to the copolymer solution to produce a final silver concentration of approximately 15%, based on the weight of coating solids in the solution.

In one embodiment, aqueous solutions of sodium chloride, zinc iodide, sodium citrate, sodium acetate, and sodium lactate (each 1.0% solutions) are added to the copolymer solution in sufficient amounts for each salt to react with 15% of the silver nitrate present in the composition. Colloids of silver chloride, silver iodide, silver citrate, silver acetate, and silver lactate are formed in the final coating composition. The coating composition also contains 25% unreacted soluble silver nitrate, as well as the silver nitrate and zinc nitrate salt products. The differences in the solubility of the different salts in the composition will result in different and prolonged rates of release of the oligodynamic silver in the coating composition when a device coated with the composition is exposed to body fluid.

In this embodiment, silver nitrate is the most soluble of the salts present in the composition and will be released rapidly upon initial exposure of the coating to body fluid. Sodium lactate, which has a lower solubility than silver nitrate but a higher solubility than the other salts present, will be released next. Then, the silver acetate, followed by the silver citrate, and then the silver chloride, and, lastly, the silver iodide will be released from the coating composition based upon their relative solubilities.

Agents can be released at various times. For example, the initial release and the duration of release of the oligodynamic agents from the composition can depend upon several factors. These factors include the relative water solubilities of the particular salts formed in the colloid and the concentration of the salts in the colloid. This release can range, for example, from a few days to several months, and can be tailored through the choice and number of salts formed in the composition for the intended purpose of the device to be coated.

Polymeric coatings can be composed of many composition. For example, the compositions of the polymeric coatings disclosed herein may contain one or more additional active agents in addition to the oligodynamic metal salts or oxides. The active agents are either retained in the composition or released from the composition at a desired rate or having a desired release profile. Nonlimiting examples of such active agents include antimicrobial agents, such as antibacterial agents, immune boosting agents, anticancer agents, angiogenic agents, polymyxins, antifungal agents, antiviral agents and antibiotics, growth factors, cytokines, immunoglobulins, pharmaceuticals, nutraceuticals, angiostatic agents, including, but not limited to, antithrombogenic agents, antitumoral agents, growth factors, antiangiogenic agents, spermicides, anesthetics, analgesics, vasodilation substances, wound healing agents, plant extracts, and other therapeutic and diagnostic agents. Other active agents may include herbicides, insecticides, algaecides, antifoulants, antifogging agents, and UV and other screening agents. The compositions can also contain salts of metals that enhance the antimicrobial effect of the oligodynamic metal, such as the platinum group metals, or other metals that promote galvanic action. The combination of additional antimicrobial compounds with oligodynamic metal compounds may provide for enhanced antimicrobial activity, for example, by resulting in synergistic antimicrobial activity.

The active agent can be present in the composition in any amount. Amounts may include from about 0.1% to about 50% of the dry weight of the composition or 1% to 30% of the composition based upon the dry weight of the composition.

The following agents have antimicrobial, antibacterial, antiviral, or antifungal activity and are examples of the types of agents that can accompany the polymer and colloid in the composition of the polymeric coatings disclosed herein. It will be understood by one of ordinary skill in the art that these are nonlimiting examples and that other active agents can be incorporated into the copolymers of the polymeric coatings disclosed herein in a manner similar to the incorporation of the specifically recited agents.

The compositions of the polymeric coatings disclosed herein can also contain additional components. For example, the compositions can contain salts of metals that enhance the antimicrobial effect of the oligodynamic metal, such as the platinum group metals, or other metals that promote galvanic action. Further, the composition can include agents that affect the release of the oligodynamic metal.

In some forms, the active agent comprises one or more biguanides, many of which have antimicrobial, antiviral, antibacterial, or antifungal activity, or some combination thereof. As used herein, the term "biguanide" includes poly (hexamethylene biguanide) hydrochloride and chlorhexidine compounds. Chlorhexidine is the term denoting the chemical compound N,N"-bis(4-chlorophenyl)-3,12-diimino-2,4,11,13-Tetraazatetrade-canediimidamide (CAS registry number 55-56-1). Chlorhexidine compounds include chlorhexidine free base as well as chlorhexidine salts, including but not limited to chlorhexidine diphosphanilate, chlorhexidine digluconate, chlorhexidine diacetate, chlorhexidine dihydrochloride, chlorhexidine dichloride, chlorhexidine dihydroiodide, chlorhexidine diperchlorate, chlorhexidine dinitrate, chlorhexidine sulfate, chlorhexidine sulfite, chlorhexidine thiosulfate, chlorhexidine di-acid phosphate, chlorhexidine difluorophosphate, chlorhexidine diformate, chlorhexidine dipropionate, chlorhexidine di-iodobutyrate, chlorhexidine di-n-valerate, chlorhexidine dicaproate, chlorhexidine malonate, chlorhexidine succinate, chlorhexidine succinamate, chlorhexidine malate, chlorhexidine tartrate, chlorhexidine dimonoglycolate, chlorhexidine mono-diglycolate, chlorhexidine dilactate, chlorhexidine di-α-hydroxyisobutyrate, chlorhexidine diglucoheptonate, chlorhexidine di-isothionate, chlorhexidine dibenzoate, chlorhexidine dicinnamate, chlorhexidine dimandelate, chlorhexidine di-isophthalate, chlorhexidine isoethionate chlorhexidine di-2-hydroxy-napthoate, and chlorhexidine embonate. Chlorhexidine salts may include the acetates, formates, gluconates, hydrochlorides, isoethionates, lactates, and succinamates of chlorhexidine. These biguanide compounds are known in the art and can be prepared by conventional methods. Numerous other biguanides are known and contemplated for use herein. Biguanides can also form polymers. Use of these biguanide polymers is also contemplated.

There are many active agents that provide antimicrobial activity. For example, Chlorhexidine is one active agent that also provides antimicrobial activity. Any effective amount of chlorhexidine can be used. In some forms, chlorhexidine is used in an amount greater than 0 and up to about 50% based on total solids in the composition by weight, or in an amount greater than 0 and up to about 10%, or in an amount between about 10% and about 50%, or in an amount between about 2 and about 10%, or in an amount between about 10% and about 20%, or in an amount between about 20% and about 30%, or in an amount between about 20% and about 30%, or in an amount between about 25% and about 50%, or in an amount between about 30% and about 40%, or in an amount between about 40% and about 50% based on total solids in the composition by weight.

In some forms, the active agent comprises one or more chlorinated phenols, many of which have antimicrobial, antibacterial, antiviral, or antifungal activity, or some combination thereof. Chlorinated phenol compounds which may be used include but are not limited to parachlorometaxylenol, dichlorometaxylenol, triclosan (2,4,4'-trichloro-2 hydroxy di-phenyl ether), 2-chlorophenol, 3-chlorophenol, 4-chlorophenol, 2,4-dichlorophenol, 2,4,6-trichlorophenol, 2,3,4,6-tetrachlorophenol, pentachlorophenol, 4-chlororesorcinol, 4,6-dichlororesorcinol, 2,4,6-trichlororesorcinol, alkylchlorophenols (including p-alkyl-o-chlorophenols, o-alkyl-p-chlorophenols, dialkyl-4-chlorophenol, and trialkyl-4-chlorophenol), dichloro-m-xylenol, chlorocresol, o-benzyl-p-chlorophenol, 3,4,6-trichlorphenol, 4-chloro-2-phenylphenol, 6-chloro-2-phenylphenol, o-benzyl-p-chlorophenol, and 2,4-dichloro-3,5-diethylphenol.

In some forms, the active agent comprises one or more quaternary ammonium compounds including but not limited to monomeric and polymeric quaternary ammonium compounds, many of which have antimicrobial, antibacterial, antiviral, or antifungal activity or some combination of the foregoing activities. Examples of quaternary ammonium compounds include, but are not limited to, benzalkonium chloride, benzethonium chloride, other benzalkonium or benzethonium halides, cetylpyridinium chloride, dequalinium chloride, N-myristyl-N-methylmorpholinium methyl sulfate, poly[N-[3-(dimethylammonio)propyl]-N'-[3-(elhyleneoxyethylene dimethy]ammonio)propyl]urea dichloride], alpha-4-[1-tris(2-hydroxyethyl)ammonium chloride-2-butenyl]-omega-tris(2-hydroxyethyl)ammonium chloride, alpha-4-[1-tris(2-hydroxyethyl)ammonium chloride-2-butenyl]poly[1-dimethyl ammonium chloride-2-butenyl]-omega-tris(2-hydroxyethyl)ammonium chloride, poly [oxy-ethylene(dimethyliminio)ethylene (dimethyliminio)-ethylene dichloride], ethyl hexadecyl dimethyl ammonium ethyl sulfate, dimethyl ammonium ethyl sulfate, dimethylethylbenzyl ammonium chloride, dimethylbenzyl ammonium chloride, and cetyldimethylethyl ammonium bromide.

In a further embodiment, the active agent comprises typical antimicrobial agents, cytokines, immunoglobulins, or pharmaceuticals and nutraceuticals. Typical active agents that are useful as antimicrobial, antiinfective, antiviral, and antibacterial agents include, but are not limited to, alexidine, aminoglycosides (such as gentamicin and Tobramycin), amoxicillin, amphotericin, ampicillin, bacitracin, beclomethasone, benzocaine, benzoic acid, beta-lactams such as pipracil and aztneonam, betamethasone, biaxin, cephalosporins such as ceftazidime, cetrimide, chloramphenicol, clarithromycin, clotrimazole, cyclosporin, docycline, erythromycin, ethylenediamine tetraacetic acid (EDTA), furazolidine, fusidic acid, gramicidin, iodine and iodine complexes such as povidone iodine and pluronic-iodine complex, macrolides, miconazole, minocycline, neomycin, nystatin, octenidine hydrochloride, ofloxacin, parachlorometaxylene, penicillin, pentoxifylline, phenolic compounds (e.g., orthophenylphenol), phenoxymethylpenicillin, picloxydine, polymixin, quinolone antibiotics (such as Norfloxacin, oxolinic acid, ciprofloxacin; Pefloxacin, Enoxacin, AM-833, Pipemidic acid and Piromidic acid, 6,8-difluoro-1-(2-fluoroethyl)-1,4-dihydro-4-oxo-7-(4-methyl-1-piperaziny-1)-quinoline-3-carboxylic acid, naladixic acid, and salts thereof) rifampicin, sorbic acid, sulfamylon, sulfonamides, tetracycline, triclocarban, vancomycins, zithromax, derivatives, metabolites, and mixtures thereof, or compounds having similar antimicrobial activity.

Some other specific examples of pharmaceutical agents that are useful as active agents include, but are not limited to, nonoxynol 9, acebutolol, acetylcysteine, acetylsalicylic acid, acyclovir, AZT, alprazolam, alfacalcidol, allantoin, allopurinol, ambroxol, amikacin, amiloride, aminoacetic acid, aminodarone, amitriptyline, amlodipine, ascorbic acid, aspartame, astemizole, atenolol, benserazide, bezafibrate, biotin, biperiden, bisoprolol, bromazepam, bromhexine, bromocriptine, budesonide, bufexamac, buflomedil, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, selegiline, chloramphenicol, chlor-pheniramine, chlortalidone, choline, cilastatin, cimetidine, cisapride, cisplatin, clavulanic acid, clomipramine, clozapine, clonazepam, clonidine, codeine, cholestyramine, cromoglycic acid, cyanocobalamin, cyproterone, desogestrel, dexamethasone, dexpanthenol, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, estradiol, ethinylestradiol, etoposide, eucalyptus globulus, famotidine, felodipine, fenofibrate, fenoterol, fentanyl, flavin mononucleotide, fluconazole, flunarizine, fluorouracil, fluoxetine, flurbiprofen, furosemide, gallopamil, gemfibrozil, gingko biloba, glibenclamide, glipizide, glycyrrhiza glabra, grapefruit seed extract, grape seed extract, griseofulvin, guaifenesin, haloperidol, heparin, hyaluronic acid, hydrochlorothiazide, hydrocodone, hydrocortisone, hydromorphone, ipratropium hydroxide, ibuprofen, imipenem, indomethacin, iohexol, iopamidol, isosorbide dinitrate, isosorbide mononitrate, isotretinoin, ketotifen, ketoconazole, ketoprofen, ketorolac, labetalol, lactulose, lecithin, levocamitine, levodopa, levoglutamide, levonorgestrel, levothyroxine, lidocaine, lipase, imipramine, lisinopril, loperamide, lorazepam, lovastatin, medroxyprogesterone, menthol, methotrexate, methyldopa, methylprednisolone, metoclopramide, metoprolol, miconazole, midazolam, minocycline, minoxidil, misoprostol, morphine, N-methylephedrine, naftidrofuryl, naproxen, nicardipine, nicergoline, nicotinamide, nicotine, nicotinic acid, nifedipine, nimodipine, nitrazepam, nitrendipine, nizatidine, norethisterone, norfloxacin, norgestrel, nortriptyline, omeprazole, ondansetron, pancreatin, panthenol, pantothenic acid, paracetamol, phenobarbital, derivatives, metabolites, and other such compounds have similar activity. It should be noted that for any term in the foregoing paragraphs that is expressed as a singular term but is sometimes interpreted as describing a class of compounds shall mean any of the group of compounds (e.g. all tetracyclines, all erythromycins, etc.).

Other pharmaceutical agents include, but are not limited to, other antibacterial, antiviral, antifungal, or antiinfective agents, antithrombogenic agents, anti-inflammatory agents, antitumoral agents, antiangiogenic agents, spermicides, anesthetics, analgesics, vasodilation substances, wound healing agents, other therapeutic and diagnostic agents, and mixtures of these.

The active agent can comprise one or more herbicide, insecticide, algaecide, antifoulant, antifogging agent, or UV or other screening agent.

The compositions of the polymeric coatings disclosed herein can contain any combination of these or other active agents. The compositions can also contain additional components such as colorants, discoloration inhibitors, agents that affect the release or rate of release of the active agent, surfactants, adhesion agents, agents that enhance the activity of the active agent, solubilizing agents, agents that enhance the lubricity of the compositions, and other agents which provide beneficial properties to the compositions.

The compositions can contain combinations of two or more of the active agents. Any combination that produces desired results may be used. Some include (along with the polymer and oligodynamic metal colloid): a combination of a biguanide (especially a chlorhexidine compound), a quaternary ammonium compound and a chlorinated phenol (for example, chlorhexidine with benzalkonium chloride and parachlorometaxylenol or triclosan); triclosan and another agent (for example ramicidin, polymixin, norfloxacin, sulfamylon, polyhexamethylene biguanide, alexidine, minocycline, iodine, benzalkonium chloride and rifampicin); chlorhexidine plus triclosan (optionally with silver sulfadiazine either as a part of the colloid or in addition to the colloid); combinations including a chlorhexidine free base and triclosan or a complex resulting from the combination of those two agents. Other examples include silver sulfadiazine (either as a part of the colloid or in addition to the colloid) and sodium piperacillin; silver sulfonamides (either as a part of the colloid or in addition to the colloid) with piperacillin; silver (either as a part of the colloid or in addition to the colloid) with a chlorinated phenol and another antiinfective or antimicrobial agent.

There are many ways to produce a polymeric coatings. For example, to produce a polymeric coating, a colloid can be formed first and then added to the polymer composition or can be formed in situ in the polymer composition. The process of forming the colloids comprises, for example, combining two or more salts, wherein at least one of the salts is the salt of an oligodynamic agent. These salts will be referred to as salt A and salt B. Salt A comprises one or more oligodynamic agents. Salt B comprises one or more salts that can react with salt A to form a colloid. Salts A and B can be combined in any amount and in any order. In some forms, salt A is present in a stoichiometric amount or in excess when compared to salt B. In some forms, salt B is present in a stoichiometric amount or in excess when compared to salt A.

Optionally, additional components can be added to the compositions. These components include, but are not limited to, additional oligodynamic agents, additional soluble salts, salts which provide galvanic action, and any other components which provide the compositions or the polymeric coatings disclosed herein with beneficial properties or enhance the antimicrobial activity of the compositions. Such components include, but are not limited to, antimicrobial agents, antibiotics, and other medicinal agents.

In one disclosed form, the composition is produced by forming a solution, dispersion, or combination of solutions and suspensions of one or more polymers. Next, a solution comprising salt A is added to the polymer composition. Then, a solution comprising salt B is added to the polymer composition to precipitate fine colloidal salt(s) of the oligodynamic agent(s) of salt A. Where the oligodynamic agent is a metal salt, the metal cation of salt A reacts with the anion of salt B. Salt B is added to the polymer composition in an amount sufficient to react with some or all of salt A. Optionally, other salts are then added in amounts to react with some or all of the remaining amount of salt A.

In another disclosed form, salt B is added to the polymer composition, followed by the addition of an excess or stoichiometric amount of salt A. In yet another form, salts A and B can be combined to form a colloid which is then added to the polymer composition.

In this embodiment, the final polymer composition formed by these processes contains one or more colloidal salts, composed of the oligodynamic cations of salt A and the anions of salt B, and one or more soluble salts, composed of the anions of salt A and the cations of salt B. Additionally, other salts may be added to the composition that do not react in solution but provide some beneficial effect such as stabilization of the colloid, modification of antimicrobial ion release rate, promotion of galvanic action, increase in antimicrobial effectiveness, or enhancement of biocompatibility. Further, other compounds may be added to the composition, including, but not limited to, medicinal agents, lubricants, nutritional agents, antioxidants, dyes and pigments, and other additives.

As noted above, any polymer can be used to form the compositions of the polymeric coatings disclosed herein. When hydrophilic polymers are used, it is preferable that the polymers be soluble in water or in organic solvents containing some water. The ability to add water to the polymer composition without precipitating the polymer allows the addition of water-soluble salts directly to the coating composition. The use of water in the polymer composition increases the solubility of the salts, resulting in the formation of finer, more stable colloids. However, it takes longer for the coating compositions to dry when the water content is very high. For this reason, in one form, the amount of water in the hydrophilic polymer compositions is about 50% or less. Such concentrations provide for faster drying times while maintaining the beneficial properties provided by the water in the composition.

In contrast, when hydrophobic polymers are used either alone or in combination with hydrophilic polymers, it is desirable to limit the amount of water present in the composition to avoid precipitation of the hydrophobic polymer with the colloid. In such instances the amount of water present in the polymer composition is preferably 1% or less. While it is possible to practice the polymeric coatings disclosed herein in the absence of water in the composition, it is preferable to have some water present. Thus, when hydrophobic polymers are employed, the water content of the polymer compositions is between about 0.1% and 1% by weight. It is advantageous to employ salts that are soluble in alcohols or organic solvents when hydrophobic polymers employed.

Examples of water-soluble silver salts suitable for use herein include, but are not limited to, silver nitrate, silver acetate and silver lactate. Persons skilled in the art will recognize that many of the "salt B" salts listed above are soluble in water and suitable for use as a water-soluble salt herein. Examples of salts which are soluble in alcohols and organic solvents include, but are not limited to, silver nitrate, sodium iodide, sodium lactate, sodium propionate, sodium salicylate, zinc chloride, zinc acetate, zinc salicylate, gold trichloride, gold tribromide, palladium chloride and hydrogen-hexachloroplatinate. Examples of alcohols that are useful in the polymeric coatings disclosed herein include, but are not limited to, methanol, ethanol, propanol, isopropanol, and butanol. Examples of organic solvents that can be used to form solutions of the oligodynamic salts include, but are not limited to, acetone, tetrahydrofuran (THF), dimethylformamide (DMF), dimethlysulfoxide (DMSO), and acetonitrile. These organic solvents are especially useful when they contain a small amount of water.

It is also possible to prepare polymer compositions from supercritical fluids. The most common of these fluids is liquefied carbon dioxide.

In one form, the polymer composition in which the colloid is formed is a hydrophilic polyether polyurethaneurea. This polymer is a substantially noncovalently crosslinked reaction product of one or more diols, water and an organic diisocyanate. The urea segments of the polymer provide improved strength, increased viscoelasticity, and decreased water absorption. These polymers typically absorb water in amounts from 50 to 100% of their weight while remaining strong and elastic.

Diols useful in the formation of these polymers include, but are not limited to, medium and long chain poly(oxyethylene) glycols having a number average molecular weights between 250 and 20,000. Examples of such diols are "Carbowax" compounds sold by Union Carbide.

Organic diisocyanates useful to form these polymers include, but are not limited to, tetramethylene diisocyanate, hexamethylene diisocyanate, trimethylhexamethylene diisocyanate, dimer acid diisocyanate, isophorone diisocyanate, diethylbenzene diisocyanate, decamethylene 1,10-diisocyanate, cyclohexylene 1,2-diisocyanate, cyclohexylene 1,4-diisocyanate, methylene bis(cyclohexyl-4-isocyanate), 2,4- and 2,6-tolylene diisocyanate, 4,4-diphenylmethane diisocyanate, 1,5-naphthaliene diisocyanate, dianisidine diisocyanate, tolidine diisocyanate, xylylene diisocyanate, and tetrahydronaphthalene-1,5-diisocyanate.

In another form, the polymer coating composition comprises a combination of a hydrophilic polyurethane, a polymer that may be similar or identical to the polymer substrate to be coated, and, optionally, other polymers which aid coating adhesion and physical properties. Antimicrobial salt colloids are prepared in this composition as disclosed previously, with the exception that, depending on the second polymer used, some or all of the water used to prepare salt solutions can be replaced with alcohols or other organic solvents to prevent precipitation of the second polymer. Another exception is that the salts elected must be soluble in solvents compatible with those in which the polymers are soluble. As an example, a solution of a hydrophilic polyether polyurethaneurea in THF can be combined with a solution of polyvinyl chloride (PVC) in methylene chloride or THF in equal amounts. Then, silver nitrate can be dissolved in ethanol and added to the solution without precipitation. Ethanol is used to dissolve the silver nitrate instead of water because PVC has a tendency to precipitate when water is added to the solution. Finally, a dilute solution of zinc chloride in ethanol/water can be slowly added to the polymer composition to produce a fine silver chloride colloid without precipitation of the PVC. The final concentration of water in the coating is less than 1%. The coating solution is then used to dip-coat PVC catheters. The finished coating is well adhered, durable, lubricious when wetted, and contains colloidal antimicrobial salts.

In another form, the polymer composition comprises a hydrophilic polymer as disclosed in U.S. Patent No. 6,329,488. In general, the polymer is a polyurethane-urea-silane copolymer prepared from the following ingredients: (1) one or more polyisocyanate, (2) one or more lubricious polymer having at least two functional groups, which may be the same or different and are reactive with an isocyanate functional group, and (3) one or more organo-functional silanes having at least two functional groups, which may be the same or different and are reactive with an isocyanate functional group and another functional group that is reactive with a silicone rubber substrate. While these copolymers may be prepared in a variety of ways, preferably they may be prepared by first forming a prepolymer from the polyisocyanate(s) and lubricious polymer(s) followed by reaction with the organo-functional silane(s). A catalyst is optionally employed during reaction of the isocyanate with the polyol.

Isocyanates useful to form these polymers include, but are not limited to, 4,4'-diphenylmethane diisocyanate and position isomers thereof, 2,4- and 2,6-toluene diisocyanate (TDI) and position isomers thereof, 3,4-dichlorophenyl diisocyanate, dicyclohexylmethane-4,4'-diisocyanate (HMDI), 4,4'-diphenylmethane diisocyanate (MDI), 1,6-hexamethylene diisocyanate (HDI) and position isomers thereof, isophorone diisocyanate (IPDI), and adducts of diisocyanates, such as the adduct of trimethylolpropane and diphenylmethane diisocyanate or toluene diisocyanate.

Polyols useful to form these polymers include, but are not limited to, polyethylene glycols, polyester polyols, polyether polyols, castor oil polyols, and polyacrylate polyols, including Desmophen A450, Desmophen A365, and Desmophen A160 (available from Mobay Corporation), poly(ethylene adipates), poly(diethyleneglycol adipates), polycaprolactone diols, polycaprolactone-polyadipate copolymer diols, poly(ethylene-terephthalate)diols, polycarbonate diols, polytetramethylene ether glycol, ethylene oxide adducts of polyoxypropylene diols, and ethylene oxide adducts of polyoxypropylene triols.

Catalysts useful to form these polymers include, but are not limited to, tertiary amines, such as N,N-dimethylaminoethanol, N,N-dimethyl-cyclohexamine-bis(2-dimethyl aminoethyl) ether, N-ethylmorpholine, N,N,N',N',N"-pentamethyl-diethylenetriamine, and 1-2(hydroxypropyl) imidazole, and metallic catalysts, such as tin, stannous octoate, dibutyl tin dilaurate, dioctyl tin dilaurate, dibutyl tin mercaptide, ferric acetylacetonate, lead octoate, and dibutyl tin diricinoleate.

Silanes useful to form these polymers include, but are not limited to, N-beta-(aminoethyl)-gamma-aminopropyl-trimethoxy silane and diamino-alkoxysilanes, such as N-(2-aminoethyl)-3-aminopropylmethyl-dimethoxy silane.

Polymers can be formed from different poritional weights. For example, these polymers preferably have from 7 to 12% by weight silane based upon the weight of the entire polymer. One ratio of isocyanate functional groups to alcohol or other isocyanate reactive functional groups is from 1.1:1 to 2:1. Viscosity of the polymer solution is a function of molecular weight of the polymer and the solids content of the solution and is controlled by addition of solvent to the solution. In one form the copolymer solution for dip coating has a kinematic viscosity in the range of about 1.5 cS to about 20 cS (centistokes), and a solids content in a range of about 0.4 to about 5.

In yet another form, the polymer composition comprises a solution of a hydrophilic polymer as defined in U.S. Pat. No. 5,290,585.

The polymer is a polyurethane-polyvinyl pyrrolidone prepared by mixing the appropriate amounts of isocyanate, polyol, and polyvinyl pyrrolidone (PVP) stock solution. Additional solvents can be added to adjust the viscosity and solids content. Solids content may be in the range of 0.4 to 15% by weight, depending on the solvent used and other considerations. The stoichiometric ratio of total NCO groups in the isocyanate to total OH groups in the polyol may vary from 0.75 to 3.0. In one form, the isocyanate has at least two NCO groups per molecule and the polyol has at least two OH groups per molecule. The ratio of polyurethane formed in situ to PVP ranges from 0.05 to 3.0 by weight.

In one embodiment, the PVP employed to form these polymers may have a mean molecular weight from about 50,000 to 2.5 million Daltons. Specific PVP polymers are Kollidon 90, Luviskol K90, Luviskol K80, and Luviskol K60, all available from BASF Corp. (Parsippany, N.J.) and Plasdone 90, PVP K90, and PVP K120, all available from GAF Corporation.

In one embodiment, isocyanates suitable to form these polymers include, but are not limited to, polymethylenepolyphenyl isocyanate, 4,4'-diphenylmethane diisocyanate and position isomers thereof, 2,4-tolylene diisocyanate and position isomers thereof, 3,4-dichlorophenyl diisocyanate, isophorone isocyanate, and adducts or prepolymers of isocyanates, such as the isocyanate prepolymer available as Vorite 63 from CasChem, Inc. (Bayonne, N.J.). Other examples of polyisocyanates having utility herein are those listed in ICI Polyurethanes Book, by George Woods, published by John Wiley and Sons, New York, N.Y. (1987).

In one embodiment, polyols useful to form these polymers include, but are not limited to, polyester polyols, polyether polyols, modified polyether polyols, polyester ether polyols, castor oil polyols, and polyacrylate polyols, including Desmophen A450, Desmophen A365, and Desmophen A160 available from Mobay Corporation (Pittsburgh, Pa.). In one form, polyols including, but not limited to, castor oil and castor oil derivatives, such as DB oil, Polycin-12, Polycin 55, and Polycin 99F, available from CasChem, Inc., may be employed. In another form, diols including, but not limited to, Desmophen 651A-65, Desmophen 1300-75, Desmophen 800, Desmophen-550 DU, Desmophen-1600U, Desmophen-1920D, and Desmophen-1150, available from Mobay Corporation, and Niax E-59 and others available from Union Carbide (Danbury, Conn.), may be employed.

Suitable solvents for use in the formation of these polymers are those which are capable of dissolving the isocyanate, the polyol, and the polyvinyl pyrrolidone without reacting with any of these components. Such solvents include, but are not limited to, methylene chloride, dibromomethane, chloroform, dichloroethane, and dichloroethylene.

In one embodiment, when a composition containing this polymeric solution is to be used as a coating, the coating is cured, after application to the substrate, at a temperature in the range of approximately 75° F. to approximately 350° F. for a period in the range of about 2 minutes to about 72 hours.

The formation of a colloid of silver chloride from silver nitrate and sodium chloride in a polyurethane polymer coating solution will now be described. It is to be understood that this is simply an example of one from of the polymeric coatings disclosed herein and that any polymer or combination of polymers and any mixture of salts that will form a colloid within the polymer solution can be employed.

In this embodiment, first, a 4.7% solution of a polyether polyurethane-urea block copolymer is prepared in a mixture of THF/ethanol in a 75/25 ratio by weight. A sufficient quantity of 10% silver nitrate (AgNO₃) solution in water is added to the CardioTech copolymer solution to produce a final silver concentration of approximately 15%, based on coating solids in the solution. An aqueous solution of 1.0% sodium chloride (NaCl) is then slowly added to the solution with stirring in an amount sufficient to react with 50% of the AgNO₃. The NaCl reacts with the AgNO₃ to produce a colloidal suspension of the poorly water soluble salt, AgCl, and the soluble salt, NaNO₃, from half of the AgNO₃. The amount of water in the final coating solution is about 30% of the total solvent weight. The final polymer concentration in the coating solution is 3.3%, based upon solvent and polymer weights.

In this embodiment, a 16 Fr latex Foley catheter can then be coated with the composition by dipping it into the composition solution, withdrawing it at a controlled rate and drying it using standard methods. The finished coating contains the water soluble, and therefore fast releasing, AgNO₃, and the water insoluble, and therefore slow releasing, AgCl.

In this embodiment, the active agent can be incorporated into the compositions of the polymeric coatings disclosed herein by any suitable method. For example, in one form, the active agent is mixed with the components of the copolymer composition in a solvent suitable for both the composition and the active agent. Such solvents include, but are not limited to, those discussed above in the process for making the composition.

In another form, the active agent or agents are mixed with the monomers that form the copolymer prior to polymerization. In this form, it is desirable that the active agent will not be deactivated by polymerization conditions and will not interfere with polymerization. The monomeric components are then polymerized by methods known in the art. In yet another form, the copolymer is formed as described above, followed by addition of the active agent to the copolymer solution.

The active agent may be soluble or insoluble in the polymer compositions of the polymeric coatings disclosed herein or may be a combination of soluble and insoluble agents. Solubilized active agents may be achieved by any means. In some forms, the active agent is first dissolved in a suitable solvent before addition to any of the solutions used to produce the compositions disclosed herein. In some forms, an active agents is solubilized by adding the dry active agent directly to a solution of the compositions disclosed herein, in which it then dissolves.

Insoluble active agents are used in some forms of the polymeric coatings disclosed herein. In one form, the active agent is dispersed into a separate solvent before addition to the solutions disclosed herein. In another form, the active agent is dispersed directly into any solution of the used to produce the compositions disclosed herein. Combinations of these techniques are also used.

As indicated above, the antimicrobial composition of the polymeric coatings disclosed herein can be used as a coating on a preformed catheter to provide antimicrobial activity to the surface of the catheter and to the environment surrounding the catheter through the continual release of oligodynamic ions. The coatings can be applied to all or part of any surface or group of surfaces on the catheter. In some forms, one or more entire surfaces of the catheter are coated. In other forms, only part of one or more surfaces is coated. In other forms, some surfaces are coated in their entirety while other surfaces are coated only partially. Partial coating may be accomplished by, for example, dipping only part of a catheter or catheter component into a coating composition or spraying a coating composition on to only a part of the catheter or catheter component.

In some forms, the compositions disclosed herein are prepared as a high solids solution and used alone or mixed with other polymers to form a catheter rather than a coating on a catheter. Polymers which are useful to form the catheters or catheter components disclosed herein include, but are not limited to, natural and synthetic rubber, especially latex rubber, acrylonitrile rubber, PVC plastisol, PVC, polyurethanes, silicone, polycarbonates, acrylates, polyamides, polypropylenes, polyethylenes, polytetrafluoroethylenes, polyvinyl acetate, poly(ethylene terephthalate), polyesters, polyamides, polyureas, styrene-block copolymers, polymethyl methacrylate, acrylic-butadiene-styrene copolymers, polystyrene, cellulose, and derivatives and copolymers of any of the above.

As nonlimiting examples, compositions disclosed herein can be admixed into latex rubber for fabrication of catheters and other dipped latex products by standard form dipping methods, and vinyl plastisols can be mixed with compositions to provide dippable and castable antimicrobial PVC devices. Thus, the final article can be composed of one or more of the compositions disclosed in admixture with other polymeric components.

Alternatively, compositions disclosed herein can be formulated into high solids coating compositions that can be used to dip-fabricate a catheter. By another method, compositions disclosed herein can be dried and melt processed, for example, by injection molding and extrusion. Compositions can be used alone or compounded with any other melt-processable material for molding and extrusion of antimicrobial articles.

When used as a coating, such as polymeric coating 48 and/or polymeric coating 50, the compositions can be applied by any means, including those methods known in the art. For example, the compositions can be brushed or sprayed onto the article, or the article can be dipped into the composition. For example, a catheter can be dipped into the antimicrobial polymer solution at a rate of 25,4 to 203,2 cm per minute (10 to 80 inches per minute), or 101,6 cm per minute (40 ipm). The catheter is allowed to remain in the antimicrobial polymer solution for a period of about 0 to 30 seconds, or about 5 to 15 seconds. The catheter is then withdrawn at a rate of about 25,4 to 203,2 cm per minute (10 to 80 ipm), or 38,1 to 76,2 cm per minute (15 to 30 ipm). Once the catheter has been coated with the copolymer disclosed herein, it is allowed to air dry for a period of at least about 10 minutes before drying is completed in an oven for a period of about 5 to 60 minutes at a temperature in the range of about 40 to 100° C. In one form, oven drying occurs for a period of about 15 minutes at a temperature of about 50° C. The coated catheter can optionally be dried with a hot air stream at a temperature in the range of approximately 40° C. to approximately 100° C. for a period of about 5 to 60 minutes to remove residual solvent. Persons skilled in the art will understand that the parameters in the foregoing paragraph are merely examples and will vary based on the composition of the substrate and coating and the desired features of the coated objects.

Variations of the amount of oligodynamic metal compounds contained in the catheter per surface area (expressed in units such as micrograms of oligodynamic metal compound per square centimeter of surface area, or µg/cm². Manipulation of this parameter provides an additional means of controlling release rate or release profile. Any achievable concentration may be used. In some forms, the article contains between about 40 and about 50 µg/cm² oligodynamic metal compound or compounds, or between about 50 and about 100 µg/cm². or between about 50 and about 75 µg/cm², or between about 25 and about 50 µg/cm², or between about 30 and about 40 µg/cm², or between about 20 and about 30 µg/cm², or between about 25 and about 30 µg/cm², or between about 10 and about 20 µg/cm². or between about 15 and about 20 µg/cm², or between about 10 and about 15 µg/cm², or between about 5 and about 15 µg/cm², or between about 5 and about 10 µg/cm², or between about 4 and about 7 µg/cm², or between about 11 and about 14 µg/cm² oligodynamic metal compound or compounds. In some forms, the article contains about 13 µg/cm² or about 8 µg/cm² oligodynamic metal compound or compounds. The foregoing ranges are obtained with coated articles as well as with articles formed from the composition.

As discussed above, in one form, the compositions disclosed herein can be coated onto the surface of a catheter or used to form a catheter. The same is true when the composition comprises one or more active agents.

It is to be understood that not necessarily all objects or advantages disclosed herein may be achieved in accordance with any particular embodiment. Thus, for example, those skilled in the art will recognize that embodiments may be carried out in a manner that achieves or optimizes one advantage or group of advantages as taught herein without necessarily achieving other objects or advantages as may be taught or suggested herein.

## Claims

1. A catheter for indwelling introduction into a body opening, comprising:
an elongated body (12) having a lumen (20) and an exterior surface, the body being formed of a polymer and being composed of or having an outside surface coated with a first oligodynamic salt;
an introducing member (32) being configured to slide along the exterior surface of said elongated body (12) and being composed of or having an outside surface coated with a second oligodynamic salt, said first oligodynamic salt and said second oligodynamic salt having different solubilities in water so that the rates of release of said first oligodynamic salt and said second oligodynamic salt are different; and
a polymeric sleeve (40) covering a substantial portion of said elongated body (12) and having a first end and a second end, said first end being secured to said introducing member (32).

2. The catheter of claim 1, wherein said elongated body (12), said introducing member (32), and said polymeric sleeve (40) are flexible, and wherein said flexible introducing member (32) is located about said exterior surface of said elongated flexible body portion (12).

3. The catheter of any one of claims 1 and 2, wherein only a portion of said introducing member (32) is configured for insertion into said body opening.

4. The catheter of any one of claims 1-3, wherein said second end of said polymeric sleeve (40) is secured to a proximal end of said elongated body (12).

5. The catheter of any one of claims 1-4, wherein the polymeric sleeve (40) is removable from said introducing member (32).

6. The catheter of any one of claims 1-5, further comprising a funnel (24) in fluid communication with said lumen (20) and positioned at a proximal end of said elongated body (12).

7. The catheter of claim 6, wherein said second end of said flexible polymeric sleeve (40) is affixed to said funnel (24).

8. The catheter of any one of claims 1-7, wherein a distal end of said elongated body (12) terminates in a tip portion.

9. The catheter of any one of claims 1-8 further comprising a tear strip in at least a portion of said sleeve (40).

10. The catheter of any one of claims 1-9 further comprising a removable cap (62) attachable to said introducing member (32).

11. The catheter of any one of claims 1-10, wherein the catheter comprises a Foley catheter.

12. The catheter of any one of claims 1-11, wherein said polymer comprises a hydrophobic polymer.

13. The catheter of any one of claims 1-12, wherein said polymer of said elongated body (12) comprises said first oligodynamic salt.

14. The catheter of any one of claims 1-12, wherein said first oligodynamic salt is a coating on at least a portion of said exterior surface of said elongated body (12).

## Patentansprüche

1. Katheter für die verweilende Einführung in eine Körperöffnung, der Folgendes umfasst:
einen länglichen Körper (12), der ein Lumen (20) und eine äußere Oberfläche aufweist, wobei der Körper aus einem Polymer ausgebildet ist und aus einer Außenoberfläche, die mit einem ersten oligodynamischen Salz beschichtet ist, besteht oder diese aufweist;
ein Einführelement (32), das zum Gleiten entlang der äußeren Oberfläche des länglichen Körpers (12) konfiguriert ist und aus einer Außenoberfläche, die mit einem zweiten oligodynamischen Salz beschichtet ist, besteht oder diese aufweist, wobei das erste oligodynamische Salz und das zweite oligodynamische Salz unterschiedliche Löslichkeiten in Wasser aufweisen, sodass die Freisetzungsraten des ersten oligodynamischen Salzes und des zweiten oligodynamischen Salzes unterschiedlich sind; und
eine Polymerhülle (40), die einen wesentlichen Teil des länglichen Körpers (12) abdeckt und ein erstes Ende und ein zweites Ende aufweist, wobei das erste Ende an dem Einführelement (32) befestigt ist.

2. Katheter nach Anspruch 1, wobei der längliche Körper (12), das Einführelement (32) und die Polymerhülle (40) flexibel sind und wobei sich das flexible Einführelement (32) um die äußere Oberfläche des länglichen flexiblen Körperteils (12) befindet.

3. Katheter nach einem der Ansprüche 1 und 2, wobei nur ein Teil des Einführelements (32) für die Einbringung in die Körperöffnung konfiguriert ist.

4. Katheter nach einem der Ansprüche 1-3, wobei das zweite Ende der Polymerhülle (40) an einem proximalen Ende des länglichen Körpers (12) befestigt ist.

5. Katheter nach einem der Ansprüche 1-4, wobei die Polymerhülle (40) von dem Einführelement (32) entfernbar ist.

6. Katheter nach einem der Ansprüche 1-5, der weiter einen Trichter (24) in Flüssigkeitsverbindung mit dem Lumen (20) umfasst, der an einem proximalen Ende des länglichen Körpers (12) positioniert ist.

7. Katheter nach Anspruch 6, wobei das zweite Ende der flexiblen Polymerhülle (40) an dem Trichter (24) angebracht ist.

8. Katheter nach einem der Ansprüche 1-7, wobei ein distales Ende des länglichen Körpers (12) in einem Spitzenteil endet.

9. Katheter nach einem der Ansprüche 1-8, der weiter einen Reiß streifen in zumindest einem Teil der Hülle (40) umfasst.

10. Katheter nach einem der Ansprüche 1-9, der weiter eine entfernbare Kappe (62) umfasst, die an das Einführelement (32) anbringbar ist.

11. Katheter nach einem der Ansprüche 1-10, wobei der Katheter einen Foley-Katheter umfasst.

12. Katheter nach einem der Ansprüche 1-11, wobei das Polymer ein hydrophobes Polymer umfasst.

13. Katheter nach einem der Ansprüche 1-12, wobei das Polymer des länglichen Körpers (12) das erste oligodynamische Salz umfasst.

14. Katheter nach einem der Ansprüche 1-12, wobei das erste oligodynamische Salz eine Beschichtung auf zumindest einem Teil der äußeren Oberfläche des länglichen Körpers (12) ist.

## Revendications

1. Cathéter d'introduction à demeure dans une ouverture corporelle, comprenant :
un corps allongé (12) ayant une lumière (20) et une face extérieure, le corps étant formé d'un polymère et étant composé d'un premier sel oligodynamique ou ayant une surface extérieure revêtue d'un premier sel oligodynamique ;
un élément d'introduction (32) étant configuré pour glisser le long de la surface extérieure dudit corps allongé (12) et étant composé d'un deuxième sel oligodynamique ou ayant une surface extérieure revêtue d'un deuxième sel oligodynamique, ledit premier sel oligodynamique et ledit deuxième sel oligodynamique ayant des solubilités différentes dans l'eau de sorte que les taux de libération dudit premier sel oligodynamique et dudit deuxième sel oligodynamique sont différents ; et
un manchon polymérique (40) couvrant une partie substantielle dudit corps allongé (12) et ayant une première extrémité et une deuxième extrémité, ladite première extrémité étant fixée solidement audit élément d'introduction (32).

2. Cathéter selon la revendication 1, dans lequel ledit corps allongé (12), ledit élément d'introduction (32), et ledit manchon polymérique (40) sont flexibles, et dans lequel ledit élément d'introduction flexible (32) est situé autour de ladite surface extérieure de ladite partie de corps allongé flexible (12).

3. Cathéter selon l'une quelconque des revendications 1 et 2, dans lequel seulement une partie dudit élément d'introduction (32) est configurée pour l'insertion dans ladite ouverture corporelle.

4. Cathéter selon l'une quelconque des revendications 1 à 3, dans lequel ladite deuxième extrémité dudit manchon polymérique (40) est fixée solidement à une extrémité proximale dudit corps allongé (12).

5. Cathéter selon l'une quelconque des revendications 1 à 4, dans lequel ledit manchon polymérique (40) peut être détaché dudit élément d'introduction (32).

6. Cathéter selon l'une quelconque des revendications 1 à 5, comprenant en outre un entonnoir (24) en communication fluidique avec ladite lumière (20) et positionné au niveau d'une extrémité proximale dudit corps allongé (12).

7. Cathéter selon la revendication 6, dans lequel ladite deuxième extrémité dudit manchon polymérique flexible (40) est fixée audit entonnoir (24).

8. Cathéter selon l'une quelconque des revendications 1 à 7, dans lequel une extrémité distale dudit corps allongé (12) se termine en une partie en pointe.

9. Cathéter selon l'une quelconque des revendications 1 à 8 comprenant en outre une bande déchirable dans au moins une partie du manchon (40).

10. Cathéter selon l'une quelconque des revendications 1 à 9 comprenant en outre un capuchon détachable (62) pouvant être attaché audit élément d'introduction (32).

11. Cathéter selon l'une quelconque des revendications 1 à 10, dans lequel le cathéter comprend un cathéter de Foley.

12. Cathéter selon l'une quelconque des revendications 1 à 11, dans lequel ledit polymère comprend un polymère hydrophobe.

13. Cathéter selon l'une quelconque des revendications 1 à 12, dans lequel ledit polymère dudit corps allongé (12) comprend ledit premier sel oligodynamique.

14. Cathéter selon l'une quelconque des revendications 1 à 12, dans lequel ledit premier sel oligodynamique est un revêtement sur au moins une partie de ladite surface extérieure dudit corps allongé (12).
